# EUROPEAN PATENT SPECIFICATION

(11) **EP 1 944 364 B1**
(45) Date of publication and mention of the grant of the patent: **12.09.2012**
(21) Application number: 06822928.5
(22) Date of filing: 02.11.2006
(51) Int. Cl.: C12N 15/10, C12Q 1/68

(54) **RNA EXTRACTION METHOD AND RNA DETECTION METHOD**
RNA-EXTRAKTIONSVERFAHREN UND RNA-NACHWEISVERFAHREN
PROCÉDÉ D'EXTRACTION DE L'ARN ET PROCÉDÉ DE DÉTECTION DE L'ARN

(30) Priority: 02.11.2005 JP 2005319332; 02.11.2005 JP 2005319333; 02.11.2005 JP 2005319334; 20.04.2006 JP 2006116310
(43) Date of publication of application: 16.07.2008
(73) Proprietor: Shimadzu Corporation, Kyoto-shi, Kyoto 604-8511 (JP)
(72) Inventor: TONOIKE, Hiroshi c/o Shimadzu Corporation, Kyoto-shi, Kyoto 604-8511 (JP); SHIRASAKI, Yoshinari c/o Shimadzu Corporation, Kyoto-shi, Kyoto 604-8511 (JP); NISHIMURA, Naoyuki c/o Shimadzu Corporation, Kyoto-shi, Kyoto 604-8511 (JP); TAMATSUKURI, Shigeru c/o Roche Diagnostics K.K., Tokyo 105-0014 (JP); WATANABE, Kuhomi c/o Roche Diagnostics K.K., Tokyo 105-0014 (JP); SAKAKURA, Yasuhiko c/o Roche Diagnostics K.K., Tokyo 105-0014 (JP); NAKAYAMA, Hiroyuki c/o Shimadzu Corporation, Kyoto-shi, Kyoto 604-8511 (JP)
(74) Representative: Wilhelms · Kilian & Partner Patentanwälte
(86) International application number: PCT/JP2006/322010
(87) International publication number: WO 2007/052765

(56) References cited:
- EP-A- 1 069 190
- WO-A-00/17320
- JP-A- 2001 029 078
- US-B1- 6 777 210
- MOSER CLAUDIO ET AL: "Isolation of functional RNA from small amounts of different grape and apple tissues." MOLECULAR BIOTECHNOLOGY FEB 2004, vol. 26, no. 2, February 2004 (2004-02), pages 95-100, XP002526690 ISSN: 1073-6085

### SEQUENCE LISTING

<110> SHIMADZU CORPORATION
<120> Method for Extracting RNA and Detecting RNA
<130> G106209 WO
<150> JP 2005-319332
   <151> 2005-11-02
<150> JP 2005-319333
   <151> 2005-11-02
<150> JP 2005-319334
   <151> 2005-11-02
<150> JP 2006-116310
   <151> 2006-04-20
<160> 5
<210> 1
   <211> 30
   <212> DNA
   <213> Artificial
<220>
   <223> synthetic primer
<400> 1
   cttcacgcag aaagcgtcta gccatggcgt 30
<210> 2
   <211> 30
   <212> DNA
   <213> Artificial
<220>
   <223> synthetic primer
<400> 2
   ctcgcaagca ccctatcagg cagtaccaca 30
<210> 3
   <211> 21
   <212> DNA
   <213> Artificial
<220>
   <223> synthetic primer
<400> 3
   actgacaatt tcatcatcac c 21
<210> 4
   <211> 21
   <212> DNA
   <213> Artificial
<220>
   <223> synthetic primer
<400> 4
   tggaattcca tcgcccactg g 21
<210> 5
   <211> 21
   <212> DNA
   <213> Artificial
<220>
   <223> synthetic primer
<400> 5
   tgtcacgatc tcatcatcac c 21

## Description

### TECHNICAL FIELD

The present disclosure relates to a method of inactivating an RNase which presents in a sample, etc.; a method of extracting RNA in a simple and stable manner from an RNA-including body (cell, fungi, bacterium, virus, and the like) which is present in the sample, or from an RNA-including body separated from the sample; a method for detecting the RNA; and a reagent used in these methods. The present disclosure relates to an RNA amplification method, and in particular, to an RNA amplification method based on Reverse Transcription-Polymerase Chain Reaction (hereinafter, abbreviated as RT-PCR).

### BACKGROUND ART

For preparing RNA used for a molecular biological analysis, it is necessary to prepare RNA in an environment where RNase does not act. Usually, it requires the steps of separating and collecting cell, fungi, bacterium, virus, and the like (hereinafter generally referred to as RNA-including body) from an object to be tested, then extracting RNA from the inside of the RNA-including body; and purifying the extracted RNA. However, RNase is unevenly distributed, and is a substance that is very difficult to be inactivated. For this reason, in purification of RNA from an RNA-including body in a biological sample, it is necessary to control RNase (suppress the activity) and remove RNase in the process of extracting RNA from inside of an RNA-including body, which requires a very strict and complicated method. Herein, as a method of conducting this process, conventionally used is a method in which a biological sample is treated with an enzyme, a surfactant, a chaotropic agent, or the like, and then RNA is extracted and purified by using, for example, phenol, phenol/chloroform or the like. In recent years, methods that use an ion exchange resin, a glass filter, glass beads, magnetic beads, or an agent having protein aggregation activity and the like, in the RNA extraction and purification step have been reported. A method of extracting and purifying RNA is described, for example, in Chomczynski & Sacchi (1987) Analytical Biochemistry, 162: 156-159. "acid guanidinium thiocyanate-phenol-chloroform extraction method :AGPC method", or in Molecular Cloning: A Laboratory Manual Third Edition (2001) Joseph. Sambrook, David W. Russell.

The RT-PCR method is a method in which after RNA is converted to complementary DNA (cDNA) using reverse transcriptase, the cDNA is amplified by the PCR method. The RT-PCR method is used as one of analytical methods realizing highest detection sensitivity and excellent quantitative ability in these days because it is able to quantitatively analyze even a trace amount of RNA. It is an essential technique, for example, for detection of virus having RNA as its gene, quantitative detection of mRNA, analysis of expressed gene by sequencing of mRNA, as well as for analysis and production of an expressed product by cloning of cDNA, and the like.

In the RT-PCR method, the PCR method carried out following the RT reaction is a method that allows amplification of a target DNA fragment as high as hundreds of thousands times by repeating DNA synthesis reactions for a specific region of DNA in a DNA chain sandwiched between primers. The PCR method is described in Japanese Unexamined Patent Publication No. 61-274697 which is the invention devised by Mullis, et al.
However, since all of RNA amplification methods including the aforementioned method is based on an enzymatic reaction, it is widely known that the reaction is strongly inhibited by pigment, protein, sugars, or unknown contaminants present in a biological sample.

Further, RNA is easily decomposed by RNase which generally presents in every biological sample.

Therefore, as described above, it is necessary to carry out a process in which cell, fungi, bacterium, virus, and the like (hereinafter, referred to as an RNA-including body) is separated from the object to be tested prior to the aforementioned amplification of RNA, and then RNA is extracted and purified from the RNA-including body. For example, U.S. Patent No. 6825340 specification PCT application WO 00/17320 and U.S. Patent No. 6777210 specification disclose inactivation of RNase by a heating treatment in the presence of a reducing agent and RNA extraction and RT-PCR from culture cells after washing with PBS. Moser et. al. (2004), Molecular Biotechnology, Vol. 26, pages 95-100, discloses a plant RNA extraction buffer with pH 9.0.
On the other hand, Japanese Unexamined Patent Publication No. 2001-29078 and European Patent Application EP 1069190 disclose direct RT-PCR from a sample comprising an RNA-including body.

As for virus detecting techniques not involving RNA amplification, Japanese Unexamined Patent Publication No. 2004-301684 discloses a diluted solution for a Norovirus specimen using an alkaline buffer agent, and detection of Norovirus by an antigen-antibody reaction using the diluted solution.
Non-patent document 1: Chomczynski and Sacchi, "Analytical Biochemistry", 1987, Vo. 162, pp. 156-159
Non-patent document 2: Joseph. Sambrook and David W. Russell, "Molecular Cloning: A Laboratory Manual Third Edition", 2001
Patent document 1: Japanese Unexamined Patent Publication No.61-274697
Patent document 2: U.S. Patent No.6825340 specification
Patent document 3: U.S. Patent No. 6777210 specification
Patent document 4: Japanese Unexamined Patent Publication No.2001-29078
Patent document 5: Japanese Unexamined Patent Publication No. 2004-301684

### DISCLOSURE OF THE INVENTION

### PROBLEMS TO BE SOLVED BY THE INVENTION

RNA is usually exposed to a risk of decomposition by RNase which generally presents not only in a biological body but also in every environment where the biological body exists. Therefore, not only conducting a rapid treatment for inactivating RNase in extraction of RNA from inside of an RNA-including body is requested, but also strict operation and management are required for preventing RNase from mixing during and after a purification process.

Even when RNA in a sample is purified by using a conventional method, however, it is often the case that removal of contaminants is difficult or an amount of recovery of RNA in sample is not constant. Particularly when a content of target RNA in a sample is small, a subsequent RNA analysis may sometimes difficult. Further, these purification methods have great chance to contamination during operation because they require complicated operations and substantial time. For this reason, conventional purification methods require skills. Accordingly, in order to solve these problems, there has been a demand for a simpler and more effective sample pretreatment method.

It is an object of the present invention to provide a method for inactivating RNase which generally presents in a sample such as biological sample, an excrement sample or an environment sample, or in a sample such as a living body-derived sample, an excrement-derived sample or an environment-derived sample obtained by separation of an RNA-including body therefrom or the like.
It is an object of the present invention to provide a method for effectively extracting virus RNA from an RNA-including body which present in a sample such as a biological sample, an excrement sample or an environment sample, or in a sample such as a living body-derived sample, an excrement-derived sample and an environment-derived sample prepared for example, by separation of an RNA-including body therefrom or the like.
It is an object of the present invention to provide a method for detecting RNA which is present in a sample in a simple, rapid and stable manner by extracting RNA effectively from the sample, and further by amplifying RNA in the sample by suppressing activity of a substance that inhibits a nucleic acid synthesis reaction.
It is an object of the present disclosure to provide a treating reagent usable in these methods.

### MEANS FOR SOLVING THE PROBLEMS

The inventors of the present invention made diligent efforts, and found that the above objects of the invention were achieved by conducting inactivation of RNase in a biological sample and extraction of RNA from inside of an RNA-including body in a single step, and subsequently conducting RNA amplification, and finally accomplished the present invention.

### <RNase inactivating methods of the disclosure>

The following description relates to an RNase inactivation method. The RNase inactivating method described below is an RNase inactivation method, in which inactivation of RNase is carried out on a sample comprising the RNase, under a heating condition by using an alkaline treating reagent comprising at least a reducing agent.

A method of inactivating RNase, comprising the steps of obtaining a mixture under the heating condition,
said mixture comprising: an RNase-comprising sample, and an alkaline treating reagent comprising at least a reducing agent, and having pH of 8.1 or higher, under a heating condition, and inactivating the RNase by keeping the mixture.

An alkaline degree of the treating reagent is such that when it is mixed with a sample to form the mixture, the mixture has pH of 8.1 or higher (in the case of 25°C). The method of inactivating RNase, wherein the treating reagent is used in a heating condition at 30°C or higher.

The method of inactivating RNase, wherein the treating reagent comprises an alkaline buffer selected from the group consisting of a Tris buffer solution, a Good buffer solution, a borate buffer solution, and a carbonate buffer solution.

The method of inactivating RNase, wherein the treating reagent comprises an alkaline substance selected from the group consisting of hydroxide, ammonia and amine.

The method of inactivating RNase, wherein the hydroxide is sodium hydroxide and/or potassium hydroxide.
The method of inactivating RNase, wherein the alkaline substance is comprised in the treating reagent at a concentration of 0.1 mM to a saturated concentration.
The method of inactivating RNase, wherein as the alkaline substance, sodium hydroxide and/or potassium hydroxide are/is comprised in the treating reagent at a concentration of 1 mM to 100 mM.

The method of inactivating RNase, wherein the reducing agent is a thiol type reducing agent.
Herein, the term thiol-type reducing agent is referred to a generic name of reducing agents having a thiol group.
The method of inactivating RNase, wherein the thiol-type reducing agent is selected from the group consisting of dithiothreitol and mercaptoethanol.
The method of inactivating RNase, wherein the reducing agent is comprised in the treating reagent at a concentration of 0.1 mM to a saturated concentration.
The method of inactivating RNase, wherein as the reducing agent, dithiothreitol is comprised in the treating reagent at a concentration of 1 mM to 100 mM.

The method of inactivating RNase, wherein the sample is selected from the group consisting of a biological sample, a living body-derived sample, an environment sample, and an environment-derived sample.

The method of inactivating RNase, wherein the sample is selected from the group consisting of an excrement sample and an excrement-derived sample.

The method of inactivating RNase, wherein the RNA-including body is selected from the group consisting of cell, fungi, bacterium and RNA virus.

The method of inactivating RNase, wherein the RNA virus is selected from the group consisting of retrovirus, norovirus (SRSV), rotavirus, and hepatitis C virus (HCV).

The method of inactivating RNase, wherein when the RNA virus is retrovirus, the retrovirus is AIDS virus (HIV).

The method of inactivating RNase, wherein the RNA is mRNA.

An RNase inactivation method comprising the steps of: mixing an RNase-comprising sample with a solution comprising at least a reducing agent; adjusting pH at 25°C of the mixture of the sample and the reducing agent to 8.1 or higher; and subjecting the mixture having adjusted pH under a heating condition to inactivate the RNase.

That is, inactivation of RNase in the above-described method is conducted by subjecting the sample to an alkaline environment having pH 8.1 or higher where the reducing agent is present.

### <RNA extraction methods of the disclosure

The following (1) to (11) relate to an RNA extraction method. That is, an extraction method of the present invention comprises conducting inactivation of RNase and extraction of RNA from an RNA-including body, on a sample comprising the RNA-including body and RNase, under a heating condition by using an alkaline treating reagent comprising at least a reducing agent and a chelate agent.
In the method of the present invention, the wordings "extraction of RNA from inside of an RNA-including body" is defined that: by breaking a membrane structure of the RNA-including body, RNA included in the membrane structure is extracted to be exposed to an outside environment of the membrane. Any other treatment conducted on the exposed RNA or outside environment in which the exposed RNA is exposed is not included in the definition of extraction in the present invention.

Disclosed herein are :
(1) An RNA extraction method, comprising the steps of:
   obtaining a mixture under a heating condition,
   said mixture comprising: a sample comprising an RNA-including body and RNase, and an alkaline treating reagent comprising at least a reducing agent, and having pH of 8.1 or higher, and
   conducting inactivation of the RNase and extraction of RNA from the RNA-including body by keeping the mixture under the heating condition.

An alkaline degree of the treating reagent is such that when it is mixed with the sample to form the mixture, the mixture has pH of 8.1 or higher (in the case of 25°C).

The RNA extraction method, wherein the heating condition is 30°C or higher.

(2) The RNA extraction method according to (1), wherein the treating reagent comprises an alkaline buffer selected from the group consisting of a Tris buffer solution, a Good buffer solution, a borate buffer solution, and a carbonate buffer solution.

(3) The RNA extraction method according to (1) or (2), wherein the treating reagent comprises an alkaline substance selected from the group consisting of hydroxide, ammonia and amine.

The RNA extraction method, wherein the hydroxide is sodium hydroxide and/or potassium hydroxide.
The RNA extraction method, wherein the alkaline substance is comprised in the treating reagent at a concentration of 0.1 mM to a saturated concentration.
The RNA extraction method, wherein as the alkaline substance, sodium hydroxide and/or potassium hydroxide are/is comprised in the treating reagent at a concentration of 1 mM to 100 mM.

(4) The RNA extraction method according to any one of (1) to (3), wherein the reducing agent is a thiol type reducing agent.

Herein, a thiol-type reducing agent is a generic name of reducing agents having a thiol group.
The RNA extraction method, wherein the thiol-type reducing agent is selected from the group consisting of dithiothreitol and mercaptoethanol.
The RNA extraction method, wherein the reducing agent is comprised in the treating reagent at a concentration of 0.1 mM to a saturated concentration.
The RNA extraction method, wherein as the reducing agent, dithiothreitol is comprised in the treating reagent at a concentration of 1 mM to 100 mM.

(5) The RNA extraction method according to any one of (1) to (4), wherein the sample is selected from the group consisting of a biological sample, a living body-derived sample, an environment sample, and an environment-derived sample.

(6) The RNA extraction method according to any one of (1) to (5), wherein the sample is selected from the group consisting of an excrement sample and an excrement-derived sample.

(7) The RNA extraction method according to any one of (1) to (6), wherein the RNA-including body is selected from the group consisting of cell, fungi, bacterium and RNA virus.

(8) The RNA extraction method according to (7), wherein the RNA virus is selected from the group consisting of retrovirus, norovirus (SRSV), rotavirus, and hepatitis C virus (HCV).

(9) The RNA extraction method according to (8), wherein when the RNA virus is retrovirus, the retrovirus is AIDS virus (HIV).

(10) The RNA extraction method according to any one of (1) to (9), wherein the RNA is mRNA.

(11) An RNA extraction method, comprising the steps of:
   mixing a sample comprising an RNA-including body and RNase in a solution comprising at least a reducing agent;
   adjusting pH at 25°C of a mixture of the sample and the reducing agent to 8.1 or higher; and
   subjecting the mixture having adjusted pH to a heating condition to conduct inactivation of the RNase and extraction of RNA from the RNA-including body.
That is, inactivation of RNase and extraction of RNA in the above methods (1) to (10) is conducted by subjecting the sample to an alkaline environment having pH of 8.1 or higher where the reducing agent is present.

### <RNA detection method>

The following (12) to (22) relate to an RNA detection method. An RNA detection method of the present disclosure comprises conducting inactivation of RNase and extraction of RNA from inside of an RNA-including body, on a sample comprising the RNA-including body and RNase, under a heating condition by using an alkaline treating reagent comprising at least a reducing agent, thereby obtaining a treated sample liquid, and conducting an RNA amplification reaction by mixing the treated sample liquid and an amplification reaction solution.
Here, the wordings "extraction of RNA from inside of an RNA-including body" is defined that: by breaking a membrane structure of the RNA-including body, RNA included in the membrane structure is taken out to be exposed to an outside environment of the membrane. Any treatment conducted on the exposed RNA and outside environment in which the exposed RNA is exposed is not included in the definition of extraction in the present invention.

The present invention relates to an RNA detection method according to appended claims 1-12. The present disclosure relates to the following :
(12) An RNA detection method, comprising the steps of:
   obtaining a mixture under a heating condition,
   said mixture comprising: a sample comprising an RNA-including body and RNase, and an alkaline treating reagent comprising at least a reducing agent, and having pH of 8.1 or higher,
   conducting inactivation of the RNase and extraction of RNA from the RNA-including body by keeping the mixture under the heating condition, thereby obtaining a treated sample liquid comprising extracted RNA, and
   conducting RNA amplification reaction by mixing the treated sample liquid and an amplification reaction solution.

The alkaline degree of the treating reagent is such that when it is mixed with the sample to form the mixture, the mixture has pH of 8.1 or higher (in the case of 25°C).

The RNA detection method, wherein the heating condition is 30°C or higher.

(13) The RNA detection method according to (12), wherein the treating reagent comprises an alkaline buffer selected from the group consisting of a Tris buffer solution, a Good buffer solution, a borate buffer solution, and a carbonate buffer solution.

(14) The RNA detection method according to (12) or (13), wherein the treating reagent comprises an alkaline substance selected from the group consisting of hydroxide, ammonia and amine.

The RNA detection method, wherein the hydroxide is sodium hydroxide and/or potassium hydroxide.
The RNA detection method, wherein the alkaline substance is comprised in the treating reagent at a concentration of 0.1 mM to a saturated concentration.
The RNA detection method, wherein as the alkaline substance, sodium hydroxide and/or potassium hydroxide are/is comprised in the treating reagent at a concentration of 1 mM to 100 mM.

(15) The RNA detection method according to any one of (12) to (14), wherein the reducing agent is a thiol type reducing agent.

Herein, a thiol-type reducing agent is a generic name of reducing agents having a thiol group.
The RNA detection method, wherein the thiol-type reducing agent is selected from the group consisting of dithiothreitol and mercaptoethanol.
The RNA detection method, wherein the reducing agent is comprised in the treating reagent at a concentration of 0.1 mM to a saturated concentration.
The RNA detection method, wherein as the reducing agent, dithiothreitol is comprised in the treating reagent at a concentration of 1 mM to 100 mM.

(16) The RNA detection method according to any one of (12) to (15), wherein the sample is selected from the group consisting of a biological sample, a living body-derived sample, an environment sample, and an environment-derived sample.

(17) The RNA detection method according to any one of (12) to (16), wherein the sample is selected from the group consisting of an excrement sample and an excrement-derived sample.

(18) The RNA detection method according to any one of (12) to (17), wherein the RNA-including body is selected from the group consisting of cell, fungi, bacterium and RNA virus.

(19) The RNA detection method according to (18), wherein the RNA virus is selected from the group consisting of retrovirus, norovirus (SRSV), rotavirus, and hepatitis C virus (HCV).

(20) The RNA detection method according to (19), wherein when the RNA virus is retrovirus, the retrovirus is AIDS virus (HIV).

(21) The RNA detection method according to any one of (12) to (20), wherein the RNA is mRNA.

The RNA detection method, wherein the mixture of the treated sample liquid and the amplification reaction solution further comprises an additive selected from the group consisting of sulfated polysaccharide, polyamine, albumin, and a nonionic surfactant.

The RNA detection method, wherein the nonionic surfactant is selected from the group consisting of polyoxyethylene sorbitan monolaurate, and a polyoxyethylene octylphenyl ether.

The RNA detection method, wherein the treating reagent further comprises sulfated polysaccharide.

(22) An RNA detection method comprising the steps of:
   mixing a sample comprising an RNA-including body and RNase in a solution comprising at least a reducing agent;
   adjusting pH at 25°C of a mixed solution of the sample and the reducing agent to 8.1 or higher;
   subjecting the mixed solution having adjusted pH, to a heating condition, to conduct inactivation of the RNase and extraction of RNA from the RNA-including body, thereby obtaining a treated sample liquid comprising extracted RNA; and
   conducting RNA amplification reaction by mixing the treated sample liquid and an amplification reaction solution.

That is, inactivation of RNase and extraction of RNA in the above methods (12) to (21) is conducted by subjecting the sample to an alkaline environment having pH of 8.1 or higher where the reducing agent is present.

### <Treating reagent>

The following relates to a treating reagent for an RNase-comprising sample.

A treating reagent for an RNase-comprising sample, comprising at least an alkaline substance and/or an alkaline buffer and a reducing agent.

A treating reagent for an RNase-comprising sample, comprising at least an alkaline substance and/or an alkaline buffer and a reducing agent, used for the above RNase inactivation method, the RNA extraction method according to any one of (1) to (11), or the RNA detection method according to any one of (12) to (22).

### EFFECT OF THE INVENTION

According to the present invention, it is possible to provide a method of inactivating RNase which generally presents in a sample such as a biological sample, an environment sample or the like, or in a sample such as a living body-derived sample obtained by separation and the like of an RNA-including body.
According to the present invention, it is possible to provide a method of extracting RNA effectively from an RNA-including body which is present in a sample such as biological sample, environment sample, or in a sample such as living body-derived sample obtained by separation and the like of an RNA-including body.
According to the present invention, by conducting inactivation of RNase in the sample and RNA extraction from inside of RNA-including body in a single step, it is possible to amplify RNA which is present in the sample in a simple, stable, effective and rapid manner. Also by suppressing activity of a substance that inhibits nucleic acid synthesis, it is possible further to amplify RNA which is present in the sample in a simple, stable, effective and rapid manner. As a result, it is possible to provide a method of detecting RNA in a sample in a simple, stable, effective and rapid manner.
According to the present disclosure, it is possible to provide a treating reagent which may be used in these methods.

### BRIEF DESCRIPTION OF THE DRAWINGS

[Fig. 1] Fig. 1 is an electrophoretogram showing a result of RNA detection in Example 1 obtained by treating a specimen in which an RNA-including body is added to human serum, with distilled water or three kinds of treating reagents having different compositions, and then conducting RNA amplification.
[Fig. 2] Fig. 2 is an electrophoretogram showing a result of RNA detection in Example 2 obtained by storing a specimen after treating with distilled water or three kinds of treating reagents having different compositions in Example 1, in cooling under refrigeration for a day, and then conducting RNA amplification.
[Fig. 3] Fig. 3 is a graph showing relationship between temperature and time of a heating treatment and an RNA detection amount, obtained in Example 3.
[Fig. 4] Fig. 4 is a graph showing relationship between heating time of a heating treatment at 85°C and an RNA detection amount, obtained in Example 4.
[Fig. 5] Fig. 5 is a graph showing relationship between temperature and time of a heating treatment and RNA detection amount, obtained in Example 5.
[Fig. 6] Fig. 6 an electrophoretogram showing a result of RNA detection in Example 8 obtained by treating a specimen in which an RNA-including body is added to human serum, with 15 kinds of treating reagents having different compositions, and then conducting RNA amplification.
[Fig. 7] Fig. 7 is an electrophoretogram showing a result of RNA detection in Example 9, obtained by treating a specimen in which an RNA-including body is added to human serum, with one of the treating reagents in Example 8, under variable heating conditions, and then conducting RNA amplification.
[Fig. 8] Fig. 8 is an electrophoretogram showing a result of RNA detection in Example 9, obtained by treating a specimen in which an RNA-including body is added to human serum, with one of the treating reagents in Example 8 further comprising EGTA, under variable heating conditions, and then conducting RNA amplification.
[Fig. 9] Fig. 9 is an electrophoretogram showing a result of RNA detection in Example 10, obtained by treating a fecal sample liquid into which a pseudo-norovirus positive fecal sample is mixed, with 8 kinds of treating reagents having compositions in which NaOH concentrations respectively differ, and then conducting RNA amplification.
[Fig. 10] Fig. 10 is an electrophoretogram showing a result of RNA detection in Example 11, obtained by treating a fecal sample liquid into which a pseudo-norovirus positive fecal sample is mixed, with 7 kinds of treating reagents having compositions in which DTT concentrations respectively differ, and then conducting RNA amplification.
[Fig. 11] Fig. 11 is an electrophoretogram showing a result of RNA detection performed on infected fecal samples having different virus concentrations without purification of RNA in Example 12.
[Fig. 12] Fig. 12 is an electrophoretogram showing a result of RNA detection performed on norovirus-infected fecal samples having different virus concentrations with purification of RNA in Example 12.
[Fig. 13] Fig. 13 is an electrophoretogram showing a result of RNA detection in Example 13 using norovirus-infected fecal samples which are respectively derived from 18 different specimens that are infected with norovirus.
[Fig. 14] Fig. 14 is an electrophoretogram showing a result of RNA detection in Example 13 using norovirus-noninfected fecal samples which are respectively derived from 10 different specimens that are not infected with norovirus.
[Fig. 15] Fig. 15 is an electrophoretogram showing a result of RNA detection in Example 14, obtained by treating a fecal sample liquid into which a pseudo-norovirus positive fecal sample is mixed, with a treating reagent in variable heating conditions, and then conducting RNA amplification.
[Fig. 16] Fig. 16 is a graph showing a result of quantification of amplified RNA by real-time PCR in Example 14.
[Fig. 17] Fig. 17 is a photograph showing appearance after mixing a model specimen with each of 15 different treating regents and conducting a heat treatment in Example 8. The upper stage shows results of using treating reagents having a DTT concentration of 0 mM, and shows the results obtained by using treating reagents [1], [2], [3], [4], [5], [6] and [7] from the left side. The lower stage shows results of using treating reagents having DTT concentration of 20 mM, and shows the results obtained by using treating reagents [8], [9], [10], [11], [12], [13], [14] and [15] from the left side.

### BEST MODE FOR CARRYING OUT THE INVENTION

The RNase inactivation method and the RNA extraction method of the present invention are realized in an alkaline environment and in the presence of a reducing agent and a chelate agent. The RNA detection method of the present invention comprises a step of conducting inactivation of RNase in a sample and RNA extraction from inside of an RNA-including body, and a step of conducting RNA amplification reaction.
A treated sample liquid obtained by the step of conducting RNase inactivation in a sample and RNA extraction from inside of an RNA-including body is directly mixed with an RNA amplification reaction solution, and subjected to RNA amplification reaction. Therefore, it is possible to directly amplify RNA from a sample without executing special purification of RNA.

### 1. Sample

The present invention may be applied to any samples to be treated insofar as the sample can contain RNase. As such a sample, a biological sample, a living body-derived sample, an environment sample, an environment-derived sample, an excrement sample, an excrement-derived sample, and the like can be exemplified.
The present invention may be particularly usefully applied when the sample to be treated contains an RNA-including body in addition to RNase. As such a sample, examples include a biological sample, a living body-derived sample, an environment sample, an environment-derived sample, an excrement sample, an excrement-derived sample, and the like. In this case, the RNase inactivation and RNA extraction from inside of the RNA-including body can be conducted in a single step.

In the present disclosure, the term "RNA-including body" means a structure surrounded by a membrane structure and having RNA inside the same. Concrete examples include cell, fungi, bacterium, virus and the like. Cells include leukocytes derived from blood or spinal fluid, oral mucosa cells and so on. Cells also include food-derived cells, detached cells from body and so on. In the present invention, when such RNA virus is comprised in the RNA-including body, RNA can be extracted and detected. As an RNA virus, retrovirus (AIDS virus (HIV) and the like), norovirus(SRSV), rotavirus, hepatitis C virus (HCV) and the like may be exemplified.

As a biological sample, examples include animal or plant tissues and body fluids and the like may be exemplified. Body fluids include a blood sample, a spinal fluid, saliva, milk and the like. Herein, blood samples include whole blood, plasma, serum and the like.
On the other hand, living body-derived samples include those obtained by conducting some sort of treatments on the above biological samples.

As an environment sample, any samples including atmospheric air, soil and water can be exemplified as far as they contain an RNA virus.
On the other hand, environment-derived sample include those obtained by conducting some sort of treatment on the environment samples.

Excrements include urine, fecal, vomitus and so on.
Excrement samples include excrement itself discharged from a living body, or those obtained by suspending excrement itself in water, saline, a pH buffer solution and the like. As a living body, examples include human, livestock, insect, and any other animals may be exemplified.
On the other hand, excrement-derived samples include samples obtained by conducting some sort of treatment on the above excrement samples.

As some sort of treatments which may be conducted on the sample, a recovery treatment of an RNA-including body may be exemplified. As a method for recovery treatment of RNA-including body, any method may be employed as far as it is able to separate an RNA-including body from the sample. For example, centrifugation or ultracentrifugation operation, filtration or ultrafiltration operation; a method of using a coprecipitation agent such as polyethylene glycol or an adsorption carrier such as antibody together with the above operation; and a method of separating by using magnetic beads, membranes and the like to which adsorption carrier is coupled are used. In any methods, the disclosed procedures are effective in the case of the recovery treatment of an RNA-including body possibly having residual RNase.

In the sample of the present invention, a substance that inhibits an RNA amplification reaction may be contained. The substance that inhibits an RNA amplification reaction is usually contained in a biological sample, a living body-derived sample, an environment sample, an environment-derived sample, an excrement sample, an excrement-derived sample, and the like. As the substance that inhibits an RNA amplification reaction, substances that may be present inside or outside cell including pigment, protein, saccharides, unknown contaminants present in a biological sample may be recited.

### 2. Treating reagent

### 2-1. pH of treating reagent-sample mixture

For conducting RNase inactivation in a sample, and RNA extraction from inside of an RNA-including body in a sample, a sample may be subjected to an alkaline environment comprising at least a reducing agent. The order of mixing or the like is irrelevant as far as an alkaline mixed solution in which at least a reducing agent and a sample are finally mixed is prepared.

Further, in the present invention, since the mixture is to be subjected to a heating condition (the item 3. below), preparing operation and heating operation of the mixture may be executed in any order. That is, the essential requirement is that the mixed solution is in such a condition that a sample is present in an alkaline solution comprising at least a reducing agent when it is subjected to a heating operation.
For example, it is permissible that one or both of the sample and the treating reagent is/are heated, and then they are mixed. In other words, it is permissible that the mixed solution is subjected to a heating condition at the same time it is prepared.
For example, it is permissible that a mixed solution of a sample and a treating reagent is prepared at room temperature and is subjected to a heating condition. In this case, the treating reagent is usually used as an aqueous solution.
Further, for example, it is permissible that a solution comprising at least a reducing agent is mixed with a sample at room temperature; pH of the obtained reducing agent-sample mixed solution is adjusted (described later); and the reducing agent-sample mixed solution having adjusted pH is subjected to a heating condition. In this case, the treating reagent itself having a composition as will be shown below is not used, however, the reducing agent-sample mixed solution having adjusted pH corresponds to the aforementioned sample-treating reagent mixture. Hereinafter, the wording of "sample-treating reagent mixture" includes this reducing agent-sample mixed solution having adjusted pH.

pH of the mixture of the treating reagent and the sample (treating reagent-sample mixture) at 25°C may be, pH 8.1 or higher, for example 8.1 to 11.1. Depending on the sample, there is a case that pH is preferably 9.0 to 11.1. As such a case, the case using an excrement sample (particularly fecal sample) or a sample derived therefrom as a sample is exemplified.

For adjusting to such an alkaline environment, an alkaline buffer and/or an alkaline substance may be contained in the treating reagent.

Examples of an alkaline buffer which may be contained in the treating reagent include, but are not limited to, Tris buffer solution, Good buffer solution, borate buffer solution, and carbonate buffer solution. Examples of buffering agent forming the Good buffer solution include, but are not limited to Tricine, MOPS, HEPES and CHES.

An alkaline substance which may be contained in the treating reagent may be selected from hydroxide, ammonia, and amine. As hydroxide, sodium hydroxide, potassium hydroxide and the like may be exemplified. As amine, tris(hydroxymethyl) aminomethane and the like may be exemplified. These may be used singly or in combination of plural kinds.

A concentration of an alkaline substance in the treating reagent is 0.1 mM to saturated concentration (saturated concentration at room temperature), and preferably 1 mM to a saturated concentration (saturated concentration at room temperature) although it differs depending on the kind of alkaline substance, kind and a concentration of a sample, and a mixing ratio with a sample.

### 2-2. Reducing agent

As a reducing agent contained in a treating reagent, a thiol-type reducing agent may be used. A thiol-type reducing agent is a generic name of reducing agents having a thiol group.
As a thiol-type reducing agent, dithiothreitol (DTT), mercaptoethanol and the like may be exemplified. Typical mercaptoethanol is 2-mercaptoethanol. These reducing agents may be used singly or in combination of plural kinds.

The concentration of the reducing agent in the treating reagent is 2.5 mM to 25 mM depending on the kind of the reducing agent, the kind and a concentration of the sample and so on.

### 2-3. Additive

The treating reagent further contains a chelate agent. It is known that hydrolysis of RNA is promoted by a bivalent metal ion. Therefore, it is effective to add a chelate agent that chelates a bivalent metal ion (EGTA, EDTA and the like) to the treating reagent.

Further, the treating reagent may further contain sulfated polysaccharide.

### 3. Heating condition

Conditions of temperature and time in the heating treatment are not particularly limited because they differ depending on difference in RNase existing amount in the sample, difference in fragility of a membrane structure resulting from difference in kind of an RNA-including body, difference in pH resulting from difference in a used amount of an alkaline substance and so like. Treating time is for example, about 1 second to 60 minutes, preferably 30 seconds to 30 minutes, and more preferably from 30 seconds to about 15 minutes.

The treating temperature is equal to or more than 60°C, for example, about 60°C or higher and not more than 100°C, preferably about 70°C to 90°C, and more preferably about 80°C to 85°C. When the treatment is executed at 80 to 85°C, the treating time may be 30 seconds to 5 minutes.

### 4. RNase inactivation and RNA extraction

By conducting a heating treatment using the above treating reagent, it is realized both of the treatments: inactivation of RNase contained in the sample, and extraction of RNA from inside the RNA-including body. Extraction of RNA occurs concurrently with, or subsequent to inactivation of RNase. In the present invention, since the both treatments can be executed by such a simple operation only by a heating treatment using the above treating reagent, it is possible to extract RNA rapidly and stably.

In inactivation of RNase, RNase is denaturated so that its enzymatic active site no longer functions. RNase is generally stable to heat, so that it is not inactivated so readily even when it is put under a heating condition. However, by the heating operation using the treating reagent according to the present invention, such RNase can be inactivated.

The wording "extraction of RNA from inside of an RNA-including body" means that a membrane structure of the RNA-including body is broken, and RNA included in the membrane structure is exposed to an environment outside the membrane. RNase which was present in the extramembrane environment of the RNA-including body is inactivated by the same treating reagent. Therefore, the risk that the exposed RNA is decomposed is dramatically reduced even though it is exposed to the extramembrane environment of the RNA-including body where it is inherently very susceptible to decomposition. Therefore, in the present invention, for achieving RNA extraction from inside of an RNA-including body, it suffices to make RNA be exposed to the extramembrane environment of the RNA-including body. This allows RNA to be present stably even if the exposed RNA is not purified immediately.

### 5. Treated sample solution

In this manner, it is possible to obtain a treated sample liquid in which RNase is inactivated and RNA is exposed. The treated sample liquid may be subjected to various steps. For example, it may be subjected to steps executed for RNA analysis, such as an RNA amplification method, a hybridization method and the like. In the treated sample liquid obtained by the method of the present invention, RNase is inactivated. Therefore, the treated sample liquid contains RNA in a stable state, and can be subjected to the above steps without necessity of any additional treatment. Of course, the treated sample liquid obtained by the method of the present invention and subjected to any other additional treatment may also be subjected to the aforementioned steps. For example, treatments for adjusting pH such as neutralization, and RNA purification treatments such as centrifugation and RNA isolation may be exemplified.

### 6. Amplification reaction solution

In the manner as described above, a treated sample liquid in which RNase is inactivated and RNA is exposed can be obtained. The obtained treated sample liquid may be used for preparation of an amplification reaction solution.
The treated sample liquid used in an amplification reaction solution may be obtained as a liquid to which no other treatment is executed after the heating treatment described above, or as a supernatant obtained by centrifugation following the heating treatment, or as a filtrate obtained by filtration.
The treated sample liquid is mixed with an RNA amplification reaction solution to form a final reaction solution. However, since the treated sample liquid is alkaline, when pH of the mixture of the treated sample liquid and the amplification reaction solution is out of a reaction condition of enzyme it is necessary to adjust pH of the mixture to fall within an optimum condition at an appropriate stage from after the heating treatment to start of amplification reaction. Optimum pH and an adjusting method of pH may be appropriately determined by those skilled in the art. As to optimum condition of pH, when such an inhibiting substance that inhibits an RNA amplification reaction during is present in a reaction system, as will be described later, it is also effective to adjust pH to alkaline region to suppress activity of the inhibiting substance. As to such optimum pH, reference may be made to Japanese Patent No. 3494509 publication and Japanese Patent No. 3452717 publication.

### 6-1. Basic composition of amplification reaction solution

As an RNA amplification reaction method, an RT-PCR method may be recited, however, any other methods for conducting RNA amplification may be used without limited to the above. Composition of the amplification reaction solution is not particularly limited, and may be appropriately determined by those skilled in the art.

When RT-PCR is executed as an RNA amplification reaction, it may be executed in the reaction form executed by preparing a mixture of a treated sample liquid and a reaction solution for RT in a tube, causing RT reaction in the tube, and adding part of the RT reaction product to a PCR reaction solution prepared in other tube, to cause a PCR reaction (Two tube-Two step); in the reaction form executed by preparing a mixture of a treated sample liquid and a RT reaction solution in a tube, causing RT reaction in the tube, and adding a reaction solution for PCR to the RT reaction product in the tube, to cause PCR reaction (One tube-Two step); and in the reaction form executed by preparing both an RT reaction solution and a PCR reaction solution in a tube, and mixing them with a treated sample liquid, to conduct RT reaction and PCR reaction sequentially (One tube-One step).

Therefore, when RT-PCR is executed, the RNA amplification reaction solution to be mixed with the treated sample liquid may be an RT reaction solution or a mixed solution of an RT reaction solution and a PCR reaction solution depending on the above execution form.

As an RT reaction solution, those known in the art may be used without any restriction. Typically, it contains a pH buffer solution, salts, primers, deoxyribonucleotides, and reverse transcriptase. As the above salts, MgCl₂, KC1 and the like are used, however, it may be changed to other salts as appropriate. By the term primer, it is meant oligonucleotide that functions as a starting point of synthesis in cDNA synthesis. Reverse transcriptase used in the RT reaction means an enzyme that is able to reversely transcript RNA to cDNA. Examples of reverse transcriptase include, but are not limited to, reverse transcriptases derived from avian retroviruses such as Rous associated virus (RAV) and Avian myeloblastosis virus (AMV); reverse transcriptases derived from murine retroviruses such as Moloney murine leukemia virus (MMLV); and Tth DNA polymerase derived from Thermus thermophilus.

As a PCR reaction solution, those known in the art may be used without any restriction. Generally, it contains a pH buffer solution, salts, primers, deoxyribonucleotides, and heat resistant DNA polymerase. As the above salts, MgCl₂, KCl or the like is used, however, it may be changed to other salts as appropriate. By the term primer, it is meant oligonucleotide that functions as a starting point of synthesis in amplification of nucleic acid. Heat resistant DNA polymerase used in PCR means a polymerase having excellent heat resistance that synthesizes DNA from primer as a base point. Examples of appropriate heat resistant DNA polymerase include, but are not limited to, *Taq* DNA polymerase derived from *Thermus aquaticus; Tth* DNA polymerase derived from *Thermus thermophilus;* KOD DNA polymerase derived from *Pyrococcus, Pfu* DNA polymerase, Pwo DNA polymerase; and mixture of these heat resistant DNA polymerases.

Since *Tth* DNA polymerase has both RT activity and PCR activity, it is advantageous in that only one kind of enzyme is required in conducting RT-PCR in One tube-One step.

### 6-2. Additive in amplification reaction solution

When a biological sample or a living body-derived sample is used as a sample comprising an RNA-including body and RNase, a treated sample liquid obtained after treatment for the above RNase inactivation and RNA extraction from inside of the RNA-including body may contain a substance that inhibits RNA amplification reaction. When such a treated sample liquid is mixed with an amplification reaction solution, the substance that inhibits the RNA amplification reaction is present in the reaction system. This may result in insufficient proceeding of the amplification reaction.

Concrete examples of a substance that inhibits an RNA amplification reaction include substances present inside or outside of cells such as for example pigments, and some kinds of protein and sugar in biological samples.

For suppressing activity of such an inhibiting substance, an additive selected from sulfated polysaccharide and polyamine may be used in the present invention.

As sulfated polysaccharide, the one selected from heparin, dextran sulfate, heparan sulfate, chondroitin sulfate, dermatan sulfate, funoran, sulfated agarose, carrageenan, porphyran, fucoidan, sulfated curdlan, and salts thereof may be used. Among these, heparin and its salt, and dextran sulfate and its salt are preferred. Sulfated polysaccharide may be used singly or in combination of several kinds.

It suffices that sulfated polysaccharide is contained in the reaction system at the time of an RNA amplification reaction. Therefore, sulfated polysaccharide may be added to either one of the treating reagent used in the heating treatment, the treated sample liquid after the heating treatment, the amplification reaction solution, and the mixture of treated sample liquid and amplification reaction solution.

As to a used amount of sulfated polysaccharide, an effective concentration range varies depending on molecular weight of sulfated polysaccharide, and existing amount of amplification reaction inhibiting substance.
For example, heparin which is one example of sulfated polysaccharide is frequently used as an anticoagulant of blood, however, it is considered to be a substance undesired for presence in a PCR reaction solution because heparin itself is known as a PCR inhibiting substance. However, when sulfated polysaccharide such as heparin is used in the present invention, as the used amount, any amount that suppresses activity of inhibiting substance of RT reaction and PCR reaction is acceptable without any particular limitation, excluding such an amount that the sulfated polysaccharide itself acts as an inhibiting substance of RT reaction and PCR reaction. As for concrete amount concerning sulfated polysaccharide, the information about sulfated polysaccharide are described in Japanese Unexamined Patent Publication No. 2000-93176.
Concretely, as a used amount of heparin, 0.1 µg/mL or more, preferably 0.3 µg/mL to 50 µg/mL is added to a final reaction solution in which a treated sample liquid and an RNA amplification reaction solution are mixed.

Polyamine is a generic name for hydrocarbon having two or more primary or secondary amino groups. Some kinds of polyamine are present in a living body, and are abundantly contained in tissues where protein and nucleic acid are actively synthesized, and have a variety of physiological activities. However, such activities are not necessary requested for polyamine in the present invention, and any polyamine may be used without particular limitation insofar as it is hydrocarbon having two or more primary or secondary amino groups in one molecule. Concrete examples of polyamine include ethylene diamine, trimethylene diamine, spermine, spermidine, diethylenetriamine, triethylenetetramine, tetraethylenepentamine and pentaethylenehexamine.

It suffices that polyamine is contained in a reaction system at the time of RNA amplification reaction. Therefore, polyamine may be added to either one of the treated sample liquid after the heating treatment, the amplification reaction solution, and the mixture of treated sample liquid and amplification reaction solution. The above information about polyamine is described in detail in Japanese Unexamined Patent Publication No.6-277061, and reference is also made to this publication for the used amount of polyamine.

In the present invention, an additive selected from albumin (Bovine Serum Albumin; BSA) and nonionic surfactant may be contained in the final reaction solution in which the treated sample liquid and the RNA amplification reaction solution is mixed. These additives may be used together with the above polyamine.

Albumin is a generic name of a group of soluble proteins contained in cells and body fluids of animals and plants. Representative examples include ovalbumin, lactalbumin in milk, serum albumin, leucosine of wheat or barley, ricin in castor bean (Ricinus communis L.) seed, and the like. Among these, serum albumin is particularly preferred, and bovine serum albumin is more preferred. Albumin is not limited to these albumins. It suffices that albumin is contained in a reaction system at the time of RNA amplification reaction. Therefore, albumin may be added to, for example, either one of a treated sample liquid after the heating treatment and pH adjustment, an amplification reaction solution, and a mixture of treated sample liquid after the heating treatment and pH adjustment and amplification reaction solution. Albumin exerts a similar effect even when it is not uniformly mixed in the final reaction solution (for example, in such a case that albumin is added to the treated sample liquid after the heating treatment and pH adjustment and then the RNA amplification reaction solution is mixed without stirring). The above information about albumin is described in detail in Japanese Unexamined Patent Publication No.2001-8685, and reference is also made to this publication for the used amount of albumin.

A nonionic surfactant is selected, for example, from polyoxyethylene sorbitan monolaurate, and polyoxyethylene octylphenyl ether. As the polyoxyethylene sorbitan monolaurate, polyoxyethylene sorbitan (20) monolaurate (Tween 20) may be exemplified. As the polyoxyethylene octylphenyl ether, polyoxyethylene (9) octylphenyl ether (Nonidet P-40(NP40)), polyoxyethylene(10) octylphenyl ether (Triton X 100) may be recited.
The above information about a nonionic surfactant is described in detail in Japanese Unexamined Patent Publication No.10-80279, and reference is also made to this publication for the used amount of a nonionic surfactant.

As a procedure of RNA amplification, after subjecting a sample to a heat treatment using the treating reagent as described above, the obtained treated sample liquid is mixed with a reaction solution and pH is adjusted as appropriate, and then amplification reaction is conducted in a known manner.
In an RT reaction, a reaction is allowed for about 30 minutes to one hour at a temperature suited for selected primers and reverse transcriptase. In PCR, a region sandwiched between primes is amplified by repeating the following three steps: a denaturation step for denaturating DNA to a single strand DNA by heat denaturation; an annealing step of causing primers sandwiching a region to be amplified to hybridize; and a polymerization step of conducting a primer extension reaction under action of DNA polymerase in the presence of deoxyribonucleotides.

### 7. Effect

According to the RNase inactivation method and the RNA extraction method of the present disclosure, since inactivation of RNase in a biological sample and RNA extraction from inside of an RNA-including body can be realized, it is possible to obtain a treated sample liquid of RNA in a simple and stable manner without purifying the RNA-including body in the biological sample. It is also expected that for the extracted RNA, influence of adsorption and embedment by contaminants such as proteins contained in the biological sample can be suppressed. Therefore, the RNA extraction method of the present disclosure is effective for subsequent detection, analysis of RNA and the like. That is, the treated sample liquid may be subjected to subsequent processes such as detection, analysis of RNA and the like without being subjected to any other treatment, or with only minimum necessary treatments such as dilution, adjustment of pH, addition of an additive and so on. Therefore, by conducting the method of the present disclosure, without worrying about influence by RNA decomposition by RNase which has been worried about at the time of conventionally executed RNA extraction, purification and the like, it is possible to conduct such a process in a simple and rapid manner. For example, the disclosed method may be used in a previous stage for RNA purification.

According to the RNA detection method of the present invention, by conducting inactivation of RNase in a biological sample and RNA extraction from inside of an RNA-including body, it is possible to amplify RNA present in the sample in a simple, stable and effective manner. Even when a substance that inhibits nucleic acid synthesis is contained in the treated sample liquid, it is possible to mitigate or suppress the activity of the inhibiting substance on nucleic acid synthesis, and amplify RNA present in the sample in a simple, stable and effective manner, for example, by dilution, by adjustment of pH, or by containment of an appropriate additive in the amplification reaction solution.

By using the present invention, it is possible to analyze foreign organisms lurking in the biological sample namely an RNA virus, retrovirus (AIDS virus (HIV) and the like), norovirus (SRSV), rotavirus, hepatitis C virus (HCV) and so on in a simple and rapid manner. Further, by using the present disclosure, it is possible to conduct analysis of expressed gene by detection of mRNA transcribed in cell or base sequencing, and analysis and production of an expressed product by cDNA cloning, in a simple and rapid manner. Further, by using the present disclosure for an environment sample such as atmospheric air, soil, water and the like, development to a microorganism test in the environment sample may be possible.

The RNA extracted by the treating reagent of the present disclosure may be stored in the treating reagent or stored after a neutralization treatment.

### Examples

In the following, the present invention will be explained in more detail by way of Examples, however, the present invention will not be limited to these Examples.

### <Example 1>

In the present example, a model specimen obtained by adding an RNA-including body to human serum (comprising RNase) was used as a sample, and to this sample, distilled water (for comparison), a NaOH aqueous solution (for comparison), a DTT aqueous solution (for comparison), or a NaOH-DTT aqueous solution serving as a treating reagent of the present invention was added, and a heating treatment was conducted, and then RNA extraction was examined.

Concretely, as an RNA-including body, Armored RNA Hepatitis C Virus (Genotype 2b) Catalog #: 42011 made by Ambion was used. A model specimen in which equivalent amounts (v/v) of human serum and an Armored RNA Hepatitis C Virus solution was mixed was prepared as a sample. Four 0.5 mL tubes each charged with 4 µL of the specimen were prepared, and each tube was added with 16 µL of (1) distilled water (for comparison), (2) 10 mM NaOH aqueous solution (for comparison), (3) 10 mM DTT aqueous solution (for comparison), or (4) aqueous solution comprising 10 mM NaOH and 10 mM DTT as a treating reagent of the present invention, respectively, and heated at 85°C for one minute.

For examining RNA extraction, RT-PCR was conducted using each treated sample liquid after a heating treatment as a template and using a primer that is specific to HCV RNA.
More specifically, immediately after the heating treatment, 1 µL of the treated sample liquid was added per 50 µL of the reaction solution, and then RT-PCR was conducted. A primer used for an RT reaction was oligonucleotide having a base sequence which is complementary to HCV RNA, and the subsequent PCR was conducted after adding oligonucleotide having a base sequence which is complementary to cDNA synthesized in the RT reaction. A product derived from RNA in the test RT-PCR is 244 bp. Sequences of used primers are as follows:
(5' primer) 5'-CTTCACGCAGAAAGCGTCTAGCCATGGCGT-3' (SEQ ID No.1)
(3' primer) 5'-CTCGCAAGCACCCTATCAGGCAGTACCACA-3' (SEQ ID No.2)

As an RT reaction solution, used was 10 mM Tris-HC1, 35 mM KCl, 1.5 mM MgCl₂, each 200 µM of dATP, dCTP, dGTP and dTTP, 2 mM DTT, 0.4 µM of 3' primer, 50 units/50 µL of Ribonuclease Inhibitor (Takara Bio, Shiga, Japan), and 5 units/50 µL of AMV XL reverse transcriptase (Takara Bio, Shiga, Japan) to which 1 mM triethylenetetramine and 0.5 µg/mL of heparin sodium were added.
RT reaction was conducted at 55°C for 30 minutes. After the reaction, reverse transcriptase was inactivated by treating at 95°C for 5 minutes.

After the RT reaction, the above RT reaction solution was added with each 20 pmol of 5' primer, and 1.25 units of *Taq* DNA polymerase (Platinum*Taq*: Invitrogen, CA, USA), and subjected to PCR.
PCR was conducted in the following manner: 2 minutes at 94°C, and 40 cycles under conditions of 30 seconds at 94°C, 30 seconds at 60°C, and 60 seconds at 72°C, and final polymerization for 7 minutes at 72°C.

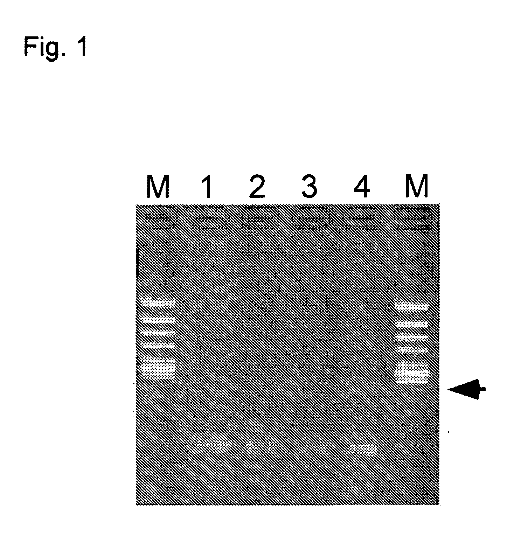

After completion of PCR, 5 µL of a reaction solution was subjected to detection by electrophoresis in a solution of 0.5 µg/mL ethidium bromide-added TAE (40 mM Tris-acetate, 1 mM EDTA) comprising 2.5% agarose. An electrophoretogram of amplification products is shown in Fig. 1. In Fig. 1, M shows the result of a size marker (250 ng of φX174-RF DNA digested by *Hinc*II), and 1, 2, 3 and 4 respectively show the results using distilled water (for comparison), 10 mM of a NaOH aqueous solution (for comparison), 10 mM of a DTT aqueous solution (for comparison), and 10 mM of a NaOH-10 mM DTT aqueous solution (treating reagent of the present invention).

Fig. 1 demonstrates that when the treating reagent of the present invention is added to the specimen (Lane 4), 244 bp of an amplification product which is specific to HCV RNA (arrow in the drawing) is obtained.

### <Example 2>

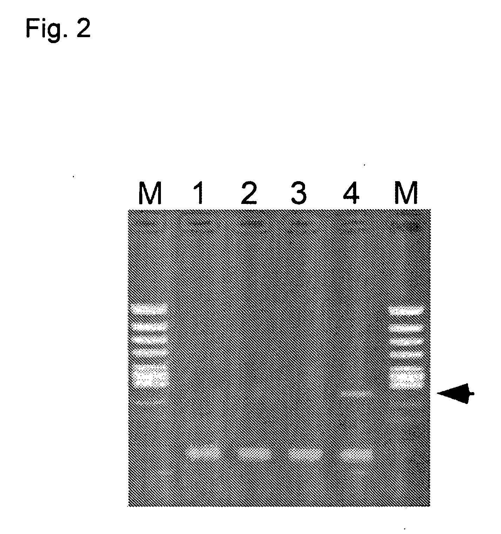

In the present example, four kinds of treated sample liquids obtained in Example 1 were subjected to RT-PCR in the same manner as in Example 1 after storing for 1 day under refrigeration, and storage stability of RNA after extraction was examined. Conditions of RT reaction, PCR reaction and electrophoresis are as same as those in Example 1. Fig. 2 shows an electrophoretogram of amplification products. In Fig. 2, M shows a result of a size marker (250 ng of φX174-RF DNA digested by *Hinc*II), 1, 2, 3 and 4 respectively show the results using distilled water (for comparison), 10 mM of a NaOH aqueous solution (for comparison), 10 mM of a DTT aqueous solution (for comparison), and 10 mM of a NaOH-10 mM DTT aqueous solution (treating reagent of the present invention).

Fig. 2 demonstrates that when the treating reagent of the present invention is used (Lane 4), 244 bp of an amplification product which is specific to HCV RNA (arrow in the drawing) is obtained even after lapse of one day from an extraction treatment. This shows that RNA after extraction by the treating reagent of the present invention exists stably.

The foregoing results of Examples 1 and 2 suggest that RNA of virus in serum can be analyzed by the treating reagent of the present invention. Therefore, it is confirmed that by using the treating reagent of the present invention, RNA can be extracted from an RNA-including body contained in a biological sample or the like by a simple operation.

### <Example 3: influence of temperature and time of heating treatment (1)>

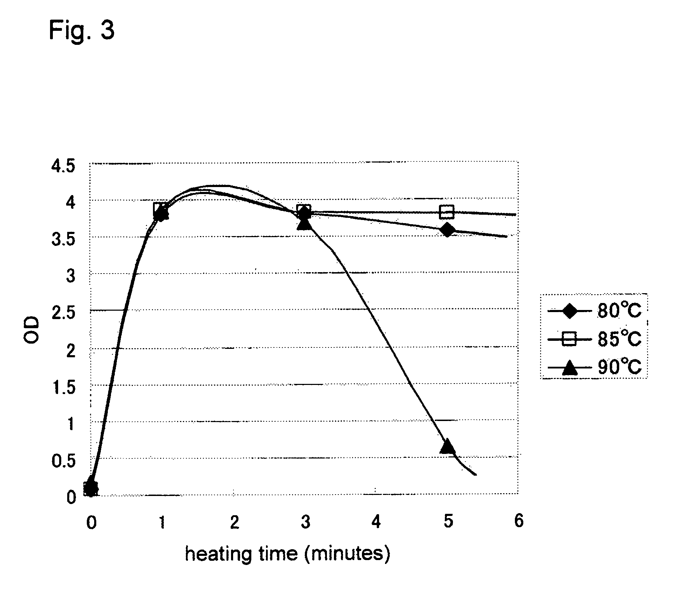

To a 1.5 mL SARSTEDT tube, 100 µL of an HCV-positive (about 100 IU/mL) plasma specimen was dispensed, 50 µL of a PEG aqueous solution (HBV SOL A supplied with "Specimen treating reagent for Amplicor^{(R)} HBV monitor" made by Roche Diagnostics K.K., the same applies also to Examples 4, 5, 6 and 7 below) was added and stirred. The mixture was centrifuged in a BenchTop microcentrifuge at 15000 rpm for 5 minutes, and the supernatant was removed. The remaining precipitate was added with 100 µL of aqueous solution comprising 12 mM of NaOH, 12 mM of DTT, and 6 µg/mL of heparin sodium as a treating reagent, stirred well on Vortex mixer, and incubated in the conditions as shown in the Table below. Immediately after heating, 50 µL of the treated sample liquid in the tube was mixed with 50 µL of Master mix of Amplicor (R) HCV v. 2.0 kit prepared in another tube, and HCV signal (OD) was measured by GeneAmp9600 (Applied Biosystems) according to a procedure of a qualitative method shown in the attached document of Amplicor^{(R)} HCV v.2.0 kit. The results are shown in Data 1 and Fig. 3. Data 1 is a table showing absorbance which is an HCV signal. In Data 1, measurements of a total of two tests are shown because replication was made. Fig. 3 is a graph showing mean value of Data 1 at each temperature, in which the vertical axis represents absorbance and the horizontal axis represents heating time.

**[Table 1]**

| Data 1 | | | | |
|---|---|---|---|---|
| | 0 min | 1 min | 3 min | 5 min |
| 80°C | 0.08 | 3.80 | 3.82 | 3.33 |
| | 0.08 | 3.82 | 3.80 | 3.82 |
| 85°C | 0.06 | 3.86 | 3.84 | 3.83 |
| | 0.08 | 3.88 | 3.83 | 3.80 |
| 90°C | 0.09 | 3.82 | 3.84 | 1.26 |
| | 0.26 | 3.86 | 3.57 | 0.06 |

As shown in Data 1, it is found that a heating treatment is effective for HCV detection by the present method. This result shows that according to the method of the present invention, RNase is inactivated, and HCV RNA is extracted from inside of HCV virus and functions as a template for RT-PCR.
As shown in Fig. 3, it is also found that RNA detection is possible by heating of about 15 seconds, and a heating time may be appropriately selected depending on the heating temperature.

### <Example 4: Influence of heating time at a temperature of heating treatment of 85°C>

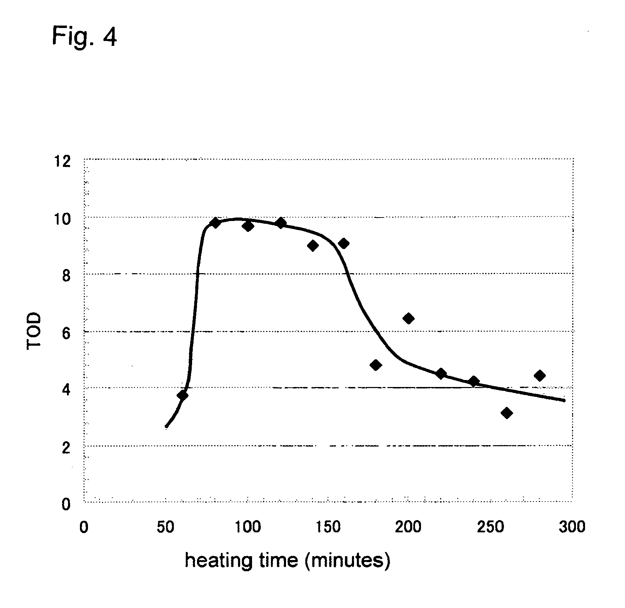

To a 1.5 mL SARSTEDT tube, 100 µL of an HCV-positive (about 1,000 IU/mL) plasma specimen was dispensed, and further 50 µL of a PEG aqueous solution was added, and stirred. This was then centrifuged by a BenchTop microcentrifuge at 15000 rpm for 5 minutes, and supernatant was removed. The remaining precipitate was added with 100 µL of an aqueous solution comprising 12 mM of NaOH, 12 mM of DTT, and 6 µg/mL of heparin sodium as a treating reagent, stirred well on Vortex mixer, and incubated at 85°C for variable time shown in the Table below. Immediately after heating, 50 µL of the treated sample liquid in the tube was mixed with 50 µL of Master mix of Amplicor^{(R)} HCV v.2.0 kit prepared in another tube, and HCV signal (total OD) was measured by GeneAmp9600 (Applied Biosystems) according to a procedure of the qualitative method shown in the attached document of Amplicor^{(R)} HCV v.2.0 kit. The results are shown in Data 2 and Fig. 4.

**[Table2]**

| Data 2 | | | | | | | | | | | | |
|---|---|---|---|---|---|---|---|---|---|---|---|---|
| Time (s) | 60 | 80 | 100 | 120 | 140 | 160 | 180 | 200 | 220 | 240 | 260 | 280 |
| TOD | 3.73 | 9.79 | 9.66 | 9.78 | 8.98 | 9.08 | 4.81 | 6.44 | 4.49 | 4.21 | 3.12 | 4.40 |

As shown in Data 2, the highest level of detection sensitivity was obtained by treatment times of 80 seconds to 160 seconds.

### <Comparative Example 1>

For inspecting effectiveness of detection method of the present invention, total OD was measured from the same specimen described in Example 4, according to the procedure of qualitative method shown in the attached document of Amplicor^{(R)} HCV v.2.0 kit. In Comparative Example, this operation was replicated five more times, and a measurement was made a total of six times. Respective measurement results were 0.75, 0.76, 1.14, 0.77, 1.30 and 1.06 shown by total OD (absorbance) which is a signal of HCV, and an average of these six measurements was 0.96.

In the method described in the attached document of Amplicor^{(R)} HCV v.2.0 kit conducted in Comparative Example 1, 1,000 µL of an RNA extraction solution is obtained from 100 µL of plasma. In the above Example 4, 100 µL of RNA extraction solution is obtained from 100 µL of plasma (in other words, an RNA extraction solution obtained in Example 4 has ten-times larger concentration). Based on the fact that total OD in Comparative Example 1 is 0.96, it can be concluded that sensitivity which is comparable to that obtained in a conventional method is obtained assuming that the total OD in Example 4 is 9.6.
In fact, in Example 4, a total OD ranging from 9 to 10 is obtained by a heating treatment time of 80 to 160 seconds. From these facts, it can be considered that the method of the present invention represented by Example 4 is able to realize sensitivity which is comparable to that obtained in a conventional method represented by Comparative Example 1.

### <Example 5: Influence by temperature and time of heating treatment (2)>

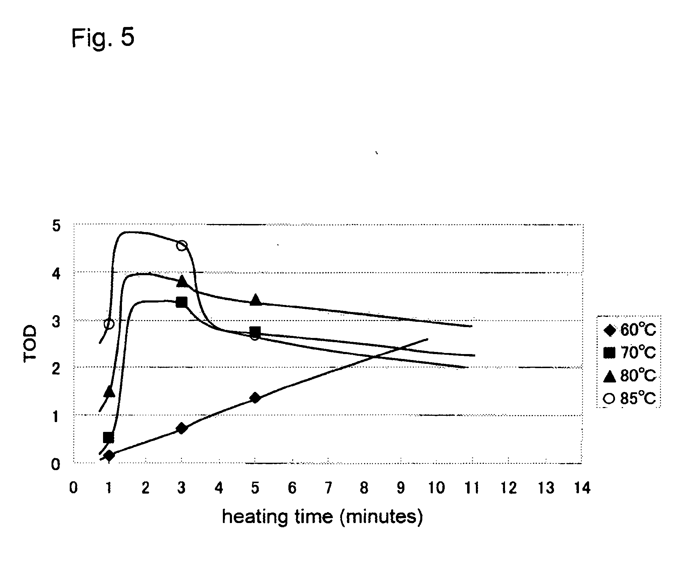

To a 1.5 mL SARSTEDT tube, 100 µL of an HCV-positive (about 1000 IU/mL) plasma specimen was dispensed, further 50 µL of a PEG aqueous solution was added and stirred. The mixture was centrifuged in a BenchTop microcentrifuge at 15000 rpm for 5 minutes, and the supernatant was removed. The remaining precipitate was added with 100 µL of an aqueous solution comprising 12 mM of NaOH, 12 mM of DTT, and 6 µg/mL of heparin sodium as a treating reagent, stirred well on a Vortex mixer, and incubated in the conditions as shown in the Table below. Immediately after heating, 50 µL of the treated sample liquid in the tube was mixed with 50 µL of Master mix of Amplicor (R) HCV v.2.0 kit prepared in another tube, and HCV signal (total OD) was measured by GeneAmp9600 (Applied Biosystems) according to a procedure of qualitative method shown in the attached document of Amplicor (R) HCV v.2.0 kit. The total OD value (integrated value of absorbance) with respect to an HCV concentration (IU/mL) is shown Data 3 below and Fig. 5. Fig. 5 is a graph showing Data 3, by representing heating time in the horizontal axis and total OD in the vertical axis. An approximation line is described based on the result of Example 4 (Fig. 4).

**[Table 3]**

| Data 3 | | | |
|---|---|---|---|
| | 1 min | 3 min | 5 min |
| 60°C | 0.16 | 0.74 | 1.37 |
| 70°C | 0.52 | 3.36 | 2.73 |
| 80°C | 1.51 | 3.82 | 3.42 |
| 85°C | 2.90 | 4.55 | 2.66 |

As shown in Data 3, it is found that even at a heating temperature of 60°C, a signal is obtained, and RNA of HCV can be detected.
As can be seen from Fig. 5, it is readily conceivable that even at a temperature of 60°C or less, RNA of HCV can be detected by heating for a long time, for example, for 5 minutes or longer. It is also readily conceivable that even at a heating temperature of higher than 85°C, RNA of HCV can be detected by heating for a short time, for example, for 30 seconds to 3 minutes.

Examples 3 to 5 demonstrate that at a heating temperature ranging from 80°C to 85°C, a stable signal having less dependency on a heating time is obtained and high sensitivity is realized. Therefore, in the conditions shown in Examples 3 to 5, it can be considered that 80°C to 85°C are particularly preferred temperature conditions. When the temperature condition is 80°C to 85°C, a heating time may be 30 seconds to 10 minutes, more preferably 30 seconds to 5 minutes, and still more preferably 80 seconds to 160 seconds.

### <Example 6>

In Example 6, each of the total of four kinds of plasma specimens including three kinds of plasma specimens which are known to be HCV-positive (about 100, 500, 5000 IU/mL) and a plasma specimen which is known to be HCV negative was added with a PEG aqueous solution, and centrifuged, and the resultant precipitates were used as samples. In the precipitate of a PEG aqueous solution from plasma, not only virus but also a lot of plasma components are present, and also RNase is present. For each sample, inactivation of RNase and RNA extraction from inside of RNA-including body were conducted according to the present invention, and RT-PCR was conducted using an HCV RNA-specific primer.

More specifically, to a 1.5 mL SARSTEDT tube, 100 µL of plasma specimen was dispensed, and further 50 µL of a PEG aqueous solution was added, and stirred. This was then centrifuged by a BenchTop microcentrifuge at 15000 rpm for 5 minutes, and supernatant was removed. The remaining precipitate was added with 100 µL of an aqueous solution comprising 12 mM of NaOH, 12 mM of DTT, and 6 µg/mL of heparin sodium as a treating reagent, stirred well on Vortex mixer, and incubated at 85°C for 2 minutes. Immediately after heating, 50 µL of the treated sample liquid in the tube was mixed with 50 µL of Master mix of Amplicor^{(R)} HCV v.2.0 kit (Roche Diagnostics K.K.) prepared in another tube, and RT-PCR was conducted by GeneAmp9600 (Applied Biosystems) according to a procedure of a qualitative method shown in the attached document of Amplicor ^{(R)} HCV v.2.0 kit. After the RT-PCR, HCV signal (total OD) was measured in a predetermined procedure according to the attached document of kit. A TOD value (integrated value of absorbance) with respect to an HCV concentration (IU/mL) is shown in Data 4 below.

**[Table 4]**

| Data 4 | | | | |
|---|---|---|---|---|
| HCVconc.(IU/ml) | 0 | 100 | 500 | 5000 |
| TOD value | 0.06 | 0.58 | 4.01 | 51.63 |

As shown in Data 4, it is found that a signal is obtained and HCV RNA can be detected in a positive specimen. This result shows that according to the method of the present invention, RNase was inactivated, and HCV RNA was extracted from inside of an HCV virus and functioned as a template for RT-PCR.
Also it demonstrates that HCV RNA is detected quantitatively because a concentration depending a TOD value of HCV is obtained.

### <Example 7>

In the present example, two kinds of specimens including a plasma specimen which is known to be HIV positive (about 700 copies/mL) and a plasma specimen which is known to be HIV negative were used. For each specimen, centrifugation by a PEG aqueous solution was conducted, and the obtained precipitate was used as a sample. For each sample, inactivation of RNase and RNA extraction from inside of an RNA-including body were conducted and RT-PCR was conducted using a HIV RNA specific primer, according to the method of the present invention.

To a 1. 5 mL SARSTEDT tube, 50 µL of plasma was dispensed, and further 25 µL of a PEG aqueous solution was added, and stirred. This was then centrifuged by a BenchTop microcentrifuge at 15000 rpm for 5 minutes, and supernatant was removed. The remaining precipitate was added with 100 µL of aqueous solution comprising 12 mM of NaOH, 12 mM of DTT, and 6 µg/mL of heparin sodium as a treating reagent, stirred well on Vortex mixer, and incubated at 85°C for 2 minutes. Immediately after heating, 50 µL of the treated sample liquid in the tube was mixed with 50 µL of Master mix of Amplicor ^{(R)} HIV v.1.5 kit (Roche Diagnostics K.K.) prepared in another tube, and RT-PCR was conducted by GeneAmp9600 (Applied Biosystems) according to a procedure of a qualitative method shown in the attached document of Amplicor ^{(R)} HIV v.1.5 kit. After the RT-PCR, HIV signal (total OD) was measured in a predetermined procedure according to the attached document of kit. A TOD value with respect to copy number/mL of HIV is shown in Data 5 below.

**[Table 5]**

| Data 5 | | |
|---|---|---|
| HIV copy number /ml | 0 | 700 |
| HIV TOD value | 0.05 | 1.10 |

As shown in Data 5, it is found that a signal is obtained and HIV RNA can be detected in a positive specimen.

In the following Examples 8 and 9, a model specimen was prepared by adding an RNA-including body to human serum (serum contains RNase). Herein, as an RNA-including body, Armored RNA Hepatitis C Virus (Genotype 2b) in TSM III Buffer Amplicor HCV Monitor Qualified Positive Control (Cat# 42011) made by Ambion Diagnostics was used. A model specimen in the present example was prepared by mixing the RNA-including body-TSM III buffer solution and human serum in a volume ratio of 1:1. A concentration of the above RNA-including body-TSM III buffer solution is defined as "73,000 IU/mL when 5(v/v)% is added to serum" and is considered as 730 IU/µL when mixed with serum in a ratio of 1:1 (volume ratio).
For RNA detection, gene amplification was first conducted by using an Amplicor HCV v2. 0 amplification reagent set (made by Roche Diagnostics K.K.). As to a temperature program of RT-PCR, a method recommended by the manufacturer was employed, and cycle number of PCR reaction was 38.
After gene amplification, detection was made by agarose electrophoresis. In the electrophoretogram, M shows a DNA size marker. As the DNA size marker, *Hinc*II digest of φX174-RF DNA was used.

### <Example 8>

The present example is an example showing that RNA in a specimen can be extracted by a treating reagent comprising an alkaline substance and a reducing agent.
Into a 200 µL plastic tube, 2 µL of a model specimen and 8 µL of each of the treating reagents (15 kinds) shown in Table 6 were employed and mixed, and heated at 85°C for 3 minutes. This was then added with 90 µL of a TE Buffer (pH 8.0), and 5 µL of the resultant mixture was mixed with 5 µL of Amplimix (7:1 mixture of HCV master mix v2.0 and an HCV manganese reagent included in Amplicor HCV v2. 0 amplification reagent set, made by Roche Diagnostics K.K.), and then RT-PCR was conducted. pH of each treating reagent (before addition of the model specimen) and pH of a model specimen-treating reagent mixture (after addition of the model specimen) are also shown in Table 6 (measured at 25°C for any cases).

**[Table 6]**

| Buffer agent | [mM] | NaOH [mM] | DTT 0mM (for comparison) | | | DTT 20mM | | |
|---|---|---|---|---|---|---|---|---|
| | | | | Before addition of model specimen | After addition of model specimen | | Before addition of model specimen | After addition of model specimen |
| HEPES | 50 | 10 | [1] | 6.8 | 7.1 | [8] | 6.9 | 7.0 |
| HEPES | 50 | 20 | [2] | 7.2 | 7.5 | [9] | 7.4 | 7.4 |
| HEPES | 50 | 40 | [3] | 8.0 | 8.3 | [10] | 8.1 | 8.1 |
| CHES | 50 | 10 | [4] | 8.8 | 8.8 | [11] | 8.7 | 8.7 |
| CHES | 50 | 20 | [5] | 9.2 | 9.3 | [12] | 9.1 | 9.0 |
| CAPS | 50 | 10 | [6] | 10.0 | 9.8 | [13] | 9.2 | 9.1 |
| CAPS | 50 | 20 | [7] | 10.4 | 10.3 | [14] | 9.7 | 9.5 |
| CAPS | 50 | 40 | | | | [15] | 10.3 | 10.1 |

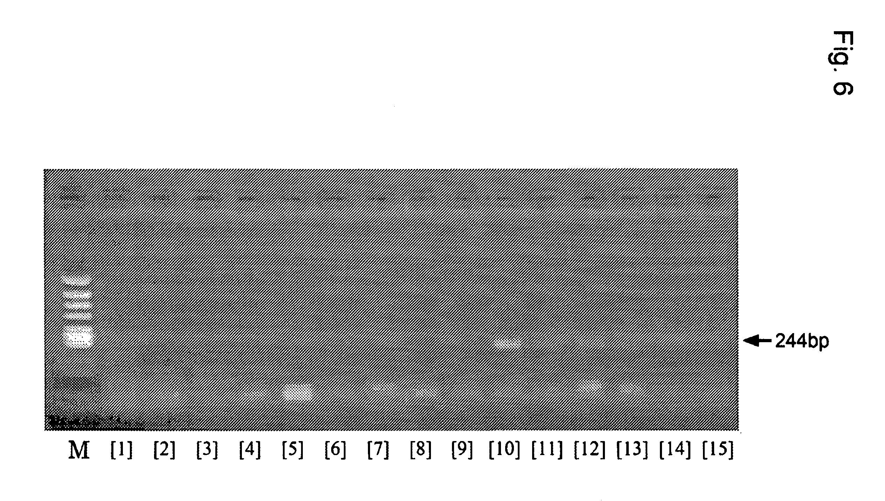

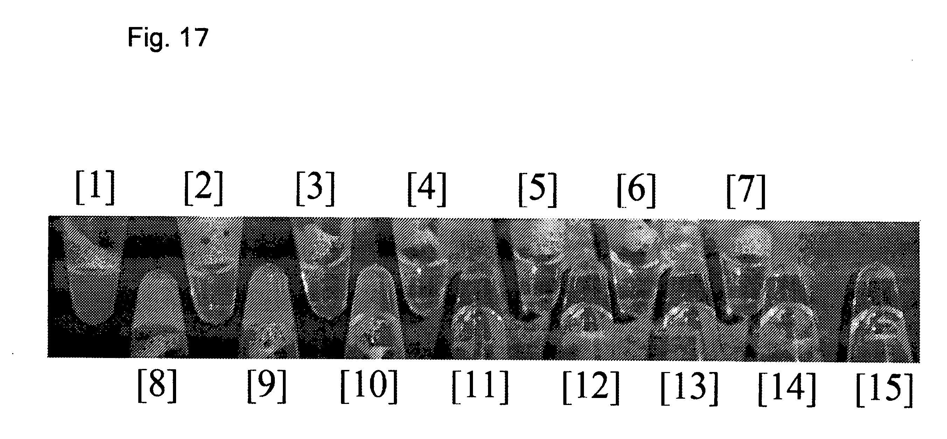

Fig. 6 shows an agarose electrophoretogram. Further Fig. 17 shows a photograph showing appearances after heating treatments in each conditions. In Fig. 17, the upper stage shows results of using treating reagents having a DTT concentration of 0 mM, and shows the results obtained by using treating reagents [1], [2], [3], [4], [5], [6] and [7] from the left side. The lower stage shows results of using treating reagents having a DTT concentration of 20 mM, and shows the results obtained by using treating reagents [8], [9], [10], [11], [12], [13], [14] and [15] from the left side.

As shown in Fig. 6, only in the case of the treating reagent comprising both of a reducing agent and alkaline, RNA was detected.
No detection in [15] may result from hydrolysis of RNA. It can be considered that hydrolysis of RNA occurs because the extracted RNA is exposed to a condition in which both a heating temperature (85°C) and pH (10.1) are high. In order to achieve the condition that is able to extract RNA effectively and to prevent hydrolysis of exposed RNA, the condition may be adjusted as follows. That is, lowering pH by adjusting a NaOH concentration, adjusting kind and a concentration of a buffer agent (preferably, the above conditions of [10] to [14]); lowering the temperature (present inventors have proved that RNA detection can be achieved without conducting heating, for example) ; or further adding a chelate agent that chelates bivalent ion such as EGTA without changing a temperature and a NaOH concentration (Example 9 below) may be conducted.

When DTT is contained, pH of the treating reagent is lowered. This is ascribable to the fact that DTT functions as acid in alkaline region.

In Fig. 17, in the conditions of a neutral region such as [8], [9], white precipitation which appears to be denatured protein was significantly observed, and it was also found that the transparency increased as pH of the used treating reagent increased. From these results, disability of detecting RNA in a neutral region may be caused by adsorption or embedment of contaminants, such as denatured protein, in RNA. On the other hand, as a cause of disability of detecting RNA in the condition of [15] may be caused by hydrolysis of RNA rather than the influence of denatured protein.

### <Example 9>

The present example demonstrates that EGTA reduces hydrolysis of RNA by heat alkaline condition. It is known that hydrolysis of RNA is promoted by a bivalent metal ion. Since some specimens intended by the present invention contain a bivalent metal ion, it is effective to add a chelate agent (EGTA and the like) that chelates such ion, to a treating reagent.

As a treating reagent, two kinds, i.e., a treating reagent [12] shown in Table 6 and a treating reagent obtained by adding 5 mM EGTA to the same treating reagent were prepared. 2 µL of a model specimen was put into a 200 µL plastic tube, and 8 µL of each treating reagent was added and mixed. At this time, as treating temperature, 25°C (for comparison), 37°C, 45°C, 50°C, 55°C, 60°C, 65°C, 70°C, 75°C, 80°C, 85°C, 90°C, 95°C, and 100°C, as a treating time, 1 minute, 3 minutes, 10 minutes, 30 minutes, and 60 minutes were examined. After the treatment, 90 µL of TE Buffer (pH 8.0) was added, and 5 µL of the resultant mixture was mixed with 5 µL of Amplimix, and subjected to RT-PCR.

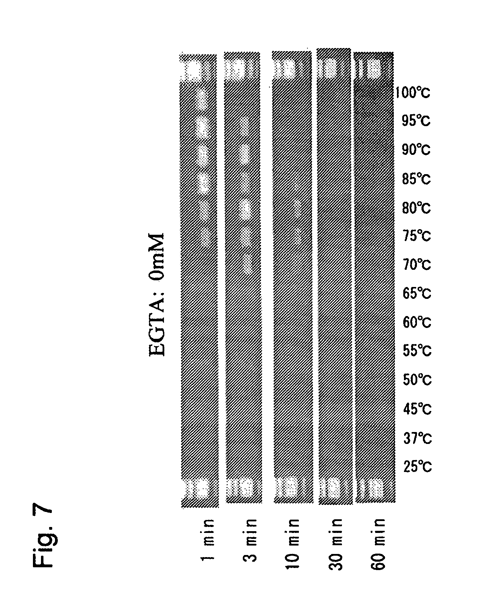

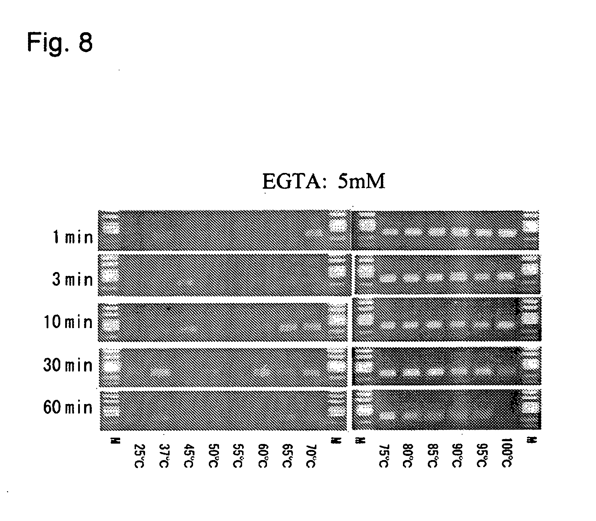

The obtained electrophoretograms are shown in Fig. 7 (EGTA concentration in the treating reagent is 0 mM) and Fig. 8 (EGTA concentration in the treating reagent is 5 mM).

As shown in Fig. 7 and Fig. 8, when EGTA was not contained in the treating reagent, almost no signal was observed after heat treatment of 30 minutes or longer. However, when EGTA was added to the treating reagent, a signal was still observed even after the heat treatment for 1 hour. It can be thought that addition of EGTA dramatically reduces the hydrolysis speed of RNA.

From the present example, it can be found that when a chelate agent that chelates a bivalent metal ion is added to the treating reagent, a temperature in the heating treatment can be set at 65°C to 100°C, more preferably 70°C to 100°C, and still preferably 70°C to 95°C.

### <Example 10>

Feces from a norovirus-negative healthy subject was suspended in saline at a concentration of 20%(w/v), and the suspension was centrifuged for 5 minutes by a microcentrifuge, and supernatant was obtained. To 198 µL of the obtained supernatant, 2 µL of a pseudo-norovirus RNA-including body (Armored RNA ^{(R)} Norwalk Virus (GenogroupII) in TSMI II Buffer: Ambion Diagnostics) was added, to prepare a pseudo-norovirus positive fecal sample liquid. 10 µL of this sample solution and 10 µL of the treating reagent were mixed in a tube so that the final liquid amount was 20 µL, and then heated at 85°C for 5 minutes. In this manner, a treated sample liquid was obtained.

In the RT-reaction of RT-PCR, 25 µL of an RT-PCR reaction solution obtained by mixing Ampdirect ^{(R)} Plus (P/N : 241-08800-98: Shimadzu Corporation), 0.4 µM of a reverse primer for pseudo-norovirus RNA (5'-ACTGACAATTTCATCATCACC-3': SEQ ID No.3), and 3.75 U AMV reverse transcriptase was mixed with 20 µL of the above treated sample liquid, and allowed to react at 42°C for 1 hour. After conducting an enzyme inactivation treatment at 95°C for 2 minutes, 0.2 µM of a forward primer for pseudo-norovirus RNA (5'-TGGAATTCCATCGCCCACTGG-3': SEQ ID No.4), and 1.25 U Nova Taq(TM) Hot Start DNA Polymerase (EMD Biosciences) were mixed in the tube after RT reaction, and the final liquid amount was made 50 µL. PCR was conducted according to a temperature program that includes preheating at 95°C for 5 minutes, 40 cycles each comprising 92°C for 30 seconds, 58°C for 30 seconds, and 72°C for 1 minute, followed by polymerization at 72°C for 7 minutes.

In Example 10, as a treating reagent, eight kinds of treating reagents A-1 to A-8 having compositions shown in Table 7 below were prepared, and the above operation was conducted on each of the eight kinds. Among these treating reagents, A-2 to A-8 are treating reagents in the present invention, and A-1 is a treating reagent prepared for comparison.

**[Table 7]**

| Treating reagent | NaOH conc. of treating reagent | DTT conc. of treating reagent | pH of treating reagent solely | pH of Treating reagent + feces (mixed ratio 1:1) |
|---|---|---|---|---|
| A-1 (for comparison) | 0mM | 20mM | 7.5 | 6.5 |
| A-2 | 10mM | 20mM | 9.1 | 8.4 |
| A-3 | 20mM | 20mM | 9.8 | 9.4 |
| A-4 | 30mM | 20mM | 10.2 | 9.9 |
| A-5 | 40mM | 20mM | 11.4 | 10.5 |
| A-6 | 50mM | 20mM | 12.0 | 11.1 |
| A-7 | 60mM | 20mM | 12.3 | 11.6 |
| A-8 | 70mM | 20mM | 12.5 | 11.8 |

Detection of PCR production was conducted using 5 µL of a reaction solution after completion of the reaction by electrophoresis in a 0.5 µg/mL ethidium bromide-added TAE (40 mM Tris-acetate, 1 mM EDTA) solution comprising 2.5% agarose gel.

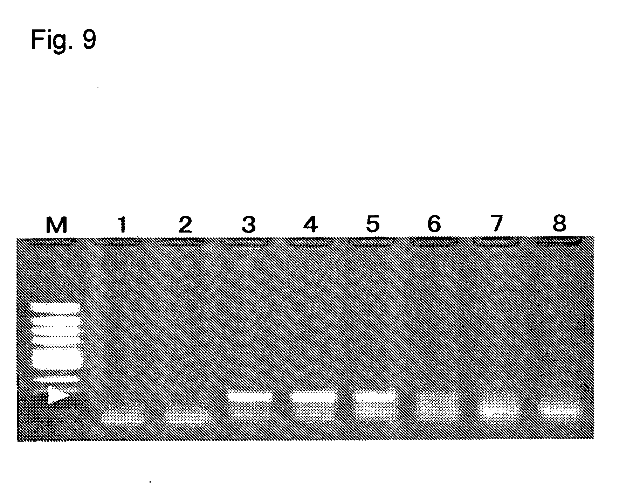

An electrophoretogram obtained by Example 10 is shown in Fig. 9. In the diagram, Lane M shows a molecular weight marker (HincII digest of φX174-RF DNA), Lane 1 represents a result of using a treating reagent A-1, Lane 2 represents a result of using a treating reagent A-2, Lane 3 represents a result of using a treating reagent A-3, Lane 4 represents a result of using a treating reagent A-4, Lane 5 represents a result of using a treating reagent A-5, Lane 6 represents a result of using a treating reagent A-6, Lane 7 represents a result of using a treating reagent A-7, and Lane 8 represents a result of using a treating reagent A-8.

### <Example 11>

Operation same as that in Example 10 was conducted except that as a treating reagent, 7 kinds of treating reagents B-1 to B-7 having the following compositions were prepared and each of such treating reagents was used. Among these treating reagents, B-2 to B-7 are treating reagents in the present invention, and B-1 is a treating reagent prepared for comparison.

B-1. 30 mM NaOH, 0 mM DTT (for comparison)
B-2. 30 mM NaOH, 5 mM DTT
B-3. 30 mM NaOH, 10 mM DTT
B-4. 30 mM NaOH, 20 mM DTT
B-5. 30 mM NaOH, 30 mM DTT
B-6. 30 mM NaOH, 40 mM DTT
B-7. 30 mM NaOH, 50 mM DTT

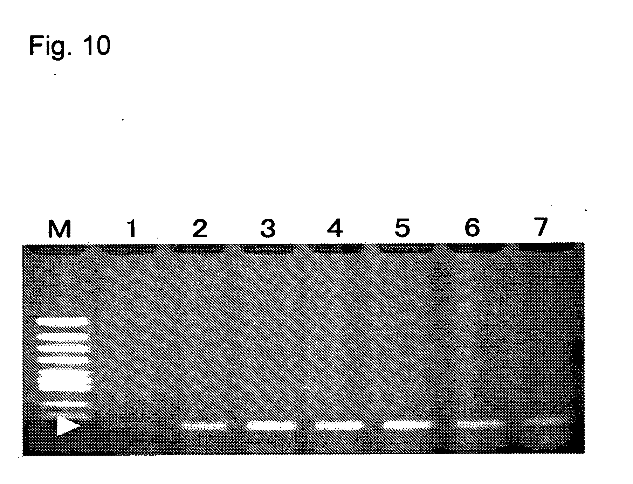

An electrophoretogram obtained by Example 11 is shown in Fig. 10. In the diagram, Lane M shows a molecular weight marker (HincII digest of φX174-RF DNA), Lane 1 represents a result of using a treating reagent B-1, Lane 2 represents a result of using a treating reagent B-2, Lane 3 represents a result of using a treating reagent B-3, Lane 4 represents a result of using a treating reagent B-4, Lane 5 represents a result of using a treating reagent B-5, Lane 6 represents a result of using a treating reagent B-6, and Lane 7 represents a result of using a treating reagent B-7.

In the above Examples 10 and 11, it can be found that NaOH and DTT are preferably contained in a treating reagent at concentrations of 20 mM to 60 mM, and 5 mM to 50 mM, respectively.

### <Example 12>

### <1> RNA detection using a fecal sample without RNA purification

Feces of a subject infected with norovirus was suspended in saline at a concentration of 20% (w/v) and centrifuged for 5 minutes by a microcentrifuge, and a supernatant was obtained.
On the other hand, feces of a norovirus-negative healthy subject was suspended in saline at a concentration of 20% (w/v) and the suspension was centrifuged for 5 minutes by a microcentrifuge, and a supernatant was obtained.
The supernatant derived from faces of infected subject was diluted by 10-fold serial dilution using the supernatant derived from faces of healthy subject, to prepare six kinds of fecal sample liquids D-1 to D-6. Concretely, a dilution ratio of D-1 is one-fold, a dilution ratio of D-2 is 10-fold, a dilution ratio of D-3 is 10²-fold, a dilution ratio of D-4 is 10³-fold, a dilution ratio of D-5 is 10⁴-fold, and a dilution ratio of D-6 is 10⁵-fold.

As a treating reagent, a treating reagent having a composition of 30 mM of NaOH, 20 mM of DTT, and 10 mM of EGTA was used.
10 µL of the above serially-diluted fecal sample liquid and 10 µL of the above treating reagent were added and stirred, and then heated at 85°C for 5 minutes.

In the RT-reaction of RT-PCR, 25 µL of an RT-PCR reaction solution obtained by mixing Ampdirect ^{(R)} Plus (P/N : 241-08800-98 : Shimadzu Corporation), 0.4 µM of a reverse primer for norovirus RNA (5'-TGTCACGATCTCATCATCACC-3': SEQ ID No. 5), and 3.75 U AMV reverse transcriptase was mixed with 20 µL of the above treated sample liquid, and allowed to react at 42°C for 1 hour. After conducting enzyme inactivation treatment at 95°C for 2 minutes, 0.2 µM of a forward primer for norovirus RNA (5'-TGGAATTCCATCGCCCACTGG-3':SEQ ID No.4), and 1.25 U Nova Taq(TM) Hot Start DNA Polymerase (EMD Biosciences) were mixed in the tube after the RT reaction, and the final liquid amount was made 50 µL. PCR was conducted according to a temperature program that includes preheating at 95°C for 5 minutes, 40 cycles each comprising 92°C for 30 seconds, 58°C for 30 seconds, and 72°C for 1 minute, followed by polymerization at 72°C for 7 minutes.

Detection of PCR production was conducted using 5 µL of a reaction after completion of the reaction by electrophoresis in a 0. 5 µg/mL ethidium bromide-added TAE (40 mM Tris-acetate, 1 mM EDTA) solution comprising 2.5% agarose gel.

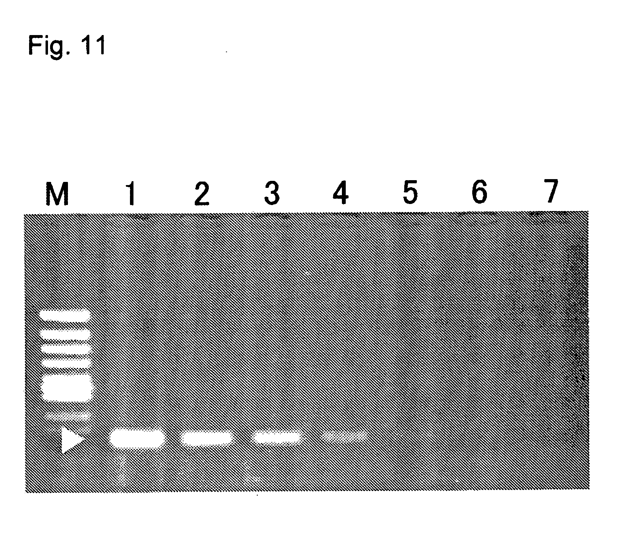

An electrophoretogram obtained by <1> is shown in Fig. 11. In the diagram, Lane 1 shows a result of using a fecal sample liquid D-1, Lane 2 shows a result of using a fecal sample liquid D-2, Lane 3 shows a result of using a fecal sample liquid D-3, Lane 4 shows a result of using a fecal sample liquid D-4, Lane 5 shows a result of using a fecal sample liquid D-5, and Lane 6 shows a result of using a fecal sample liquid D-6. Lane 7 shows Negative Control, that is, a result obtained by conducting the same operation except that feces of a norovirus-uninfected healthy subject is used in place of feces of a norovirus-infected subject. Lane M is a molecular weight marker (HincII digest of φX174-RF DNA).

### <2> RNA detection using RNA purified from fecal sample

To each diluted supernatant derived from feces of infected subject obtained in the above <1>, QIAamp Viral RNA Mini Kit (QIAGEN) was applied to purify RNA, thereby obtaining six kinds of purified RNA liquids E-1 to E-6, which were controls for fecal sample liquids D-1 to D-6 in the above <1>. Concretely, E-1 is a purified RNA liquid corresponding to D-1 (1-fold), E-2 is a purified RNA liquid corresponding to D-2 (10-fold), E-3 is a purified RNA liquid corresponding to D-3 (10²-fold), E-4 is a purified RNA liquid corresponding to D-4 (10³-fold), E-5 is a purified RNA liquid corresponding to D-5 (10⁴-fold), and E-6 is a purified RNA liquid corresponding to D-6 (10⁵-fold).

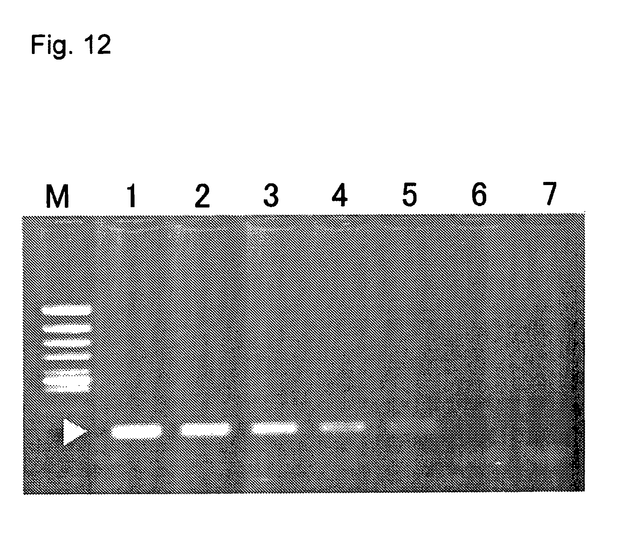

The same operation as in the above <1> was conducted except that purified RNA liquids E-1 to E-6 were respectively used in place of the fecal sample liquids D-1 to D-6.
An electrophoretogram obtained by <2> is shown in Fig. 12. In the diagram, Lane 1 shows a result of using a purified RNA liquid E-1, Lane 2 shows a result of using a purified RNA liquid E-2, Lane 3 shows a result of using a purified RNA liquid E-3, Lane 4 shows a result of using a purified RNA liquid E-4, Lane 5 shows a result of using a purified RNA liquid E- 5, and Lane 6 shows a result of using a purified RNA liquid E-6. Lane 7 shows Negative Control, that is, a result obtained by conducting the same operation except that feces of a norovirus-uninfected healthy subject was used in place of feces of a norovirus-infected subject. Lane M is a molecular weight marker (*Hinc*II digest of φX174-RF DNA).

Based on Fig. 11 and Fig. 12, sensitivity and specificity of RNA detection were compared between the case where a fecal sample was used without RNA purification and the case where RNA purified from a fecal sample was used. A detection limit was at a dilution ratio of 10⁴-fold for both samples.

In fecal supernatant, not only viruses but also a lot of bacteria and living body-derived substances are floating, and RNase is also present abundantly in such supernatant. The results shown by the above example can be interpreted that by practice of the present invention, inactivation of RNase which is abundantly present in feces and RNA extraction from RNA virus, and further control of an RT-PCR inhibiting substance effectively operate.

### <Example 13>

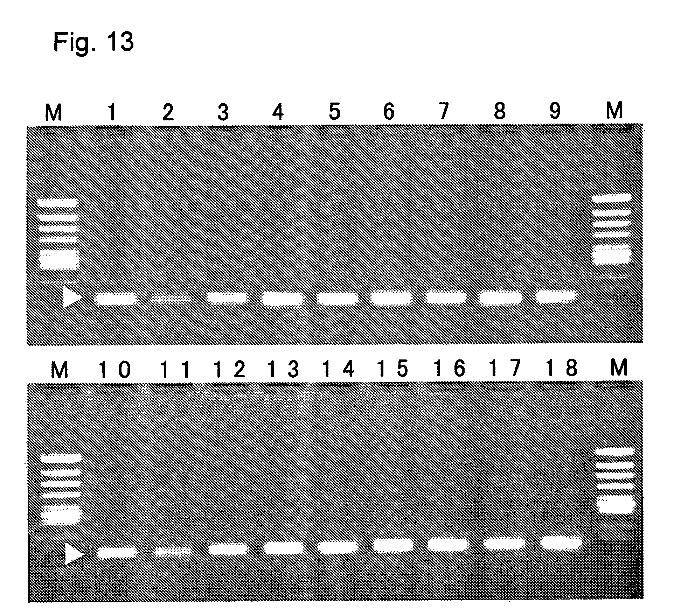

The operation same as that described in <1> of Example 12 was conducted on 18 kinds of norovirus-positive feces respectively derived from 18 different specimens infected with norovirus (specimen numbers 1 to 18). The obtained electrophoretogram is shown in Fig. 13. In Fig. 13, the numbering of lanes corresponds to the number of specimen. Lane M is a molecular weight marker (*Hinc*II digest of φX174-RF DNA).

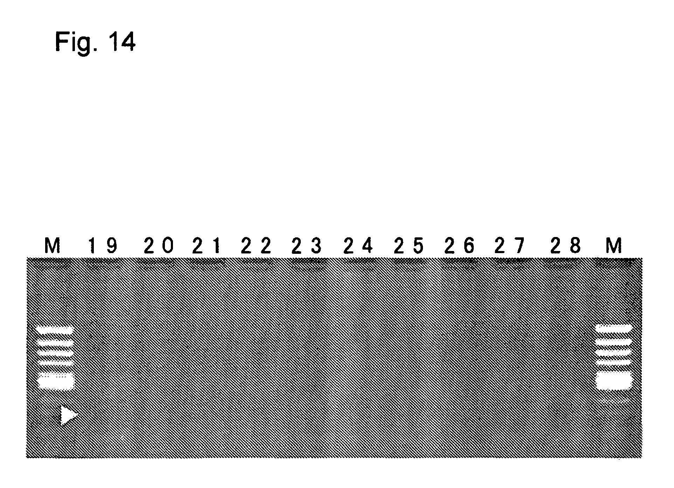

On the other hand, the operation same as that described in <1> of Example 12 was conducted on 10 kinds of norovirus-negative feces respectively derived from 10 different specimens not infected with norovirus (specimen numbers 19 to 28). The obtained electrophoretogram is shown in Fig. 14. In Fig. 14, the numbering of lanes corresponds to the number of specimen. Lane M is a molecular weight marker (HincII digest of φX174-RF DNA).

As shown in Fig. 13 and Fig. 14, from the norovirus-positive fecal samples, a specific product was detected in every case (18 from 18 specimens). On the other hand, from the norovirus-negative fecal samples, a mimic product was not detected in every case (10 from 10 specimens).

### <Example 14>

Operation was conducted in the same manner as described in Example 10 except that as a treating reagent, a treating reagent having a composition of 30 mM NaOH, 20 mM DTT and 10 mM EGTA was used, and the heating treatment was conducted at various temperatures ranging from 20°C to 100°C in various time conditions ranging from 1 minutes to 60 minutes.

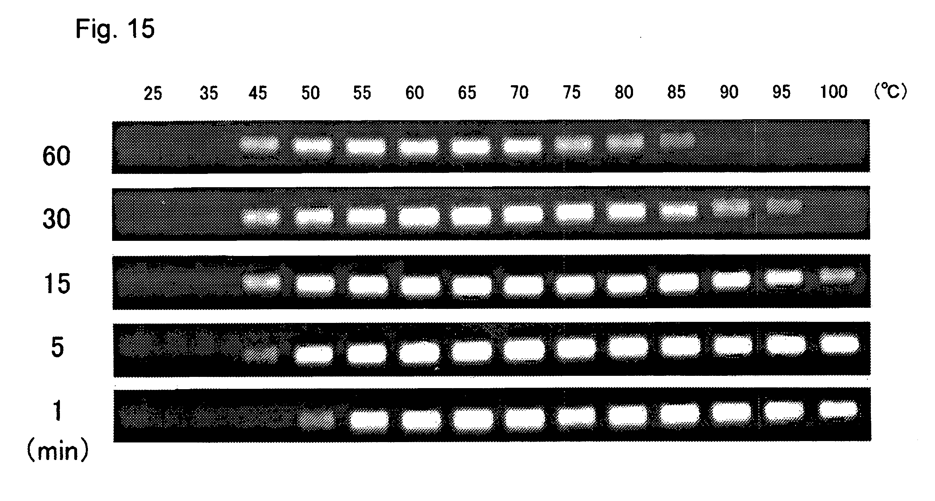

An electrophoretogram obtained by Example 14 is shown in Fig. 15. In Fig. 15, five lanes respectively correspond to the cases of a heating treatment time of 1 min., 5 min., 15 min., 30 min., and 60 min., and for each lane, the results of the cases where a temperature in the heating treatment is 25°C (for comparison), 35°C, 45°C, 50°C, 55°C, 60°C, 65°C 70°C, 75°C, 80°C, 85°C, 90°C, 95°C, and 100°C are shown.

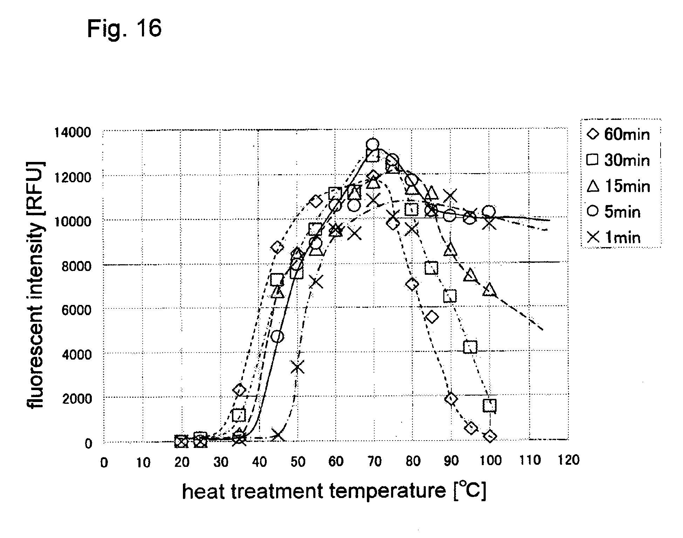

Amplified RNA was quantified by real-time PCR. Concretely, to the obtained RT-PCR reaction solution, 10×SYBR(TM) Green I (Molecular Probes) was added, and a temperature program that includes preheating at 95°C for 5 minutes, 30 cycles each comprising 92°C for 30 seconds, 58°C for 30 seconds, and 72°C for 1 minute, followed by polymerization at 72°C for 7 minutes was executed. Fluorescent intensity at 30-th cycles is shown in Fig. 16. In Fig. 16, the horizontal axis represents a heat treatment temperature (°C), and the vertical axis represents fluorescent intensity (relative fluorescent intensity: RFU).

Also in this example, it can be considered that by adding EGTA, hydrolysis speed of RNA is significantly reduced, which enables stable RNA detection in wide range of heating conditions.

In the above-described Examples, concrete forms in the scope of the present invention have been shown, however, the present invention may be practiced in various other forms without limited to these Examples. Therefore, the above-described Examples are merely exemplification in all respects, and should not be interpreted in a limitative manner.
In the sequencing list free text (Description of Artificial Sequence), SEQ ID Nos. 1 to 5 are synthetic primers.

### Industrial applicability

According to the present disclosure, it is possible to provide a method of inactivating RNase which generally presents in a sample such as a biological sample and an environment sample, or in a sample such as a living body-derived sample obtained by separation of an RNA-including body and the like.
According to the present disclosure, it is possible to provide a method of extracting RNA effectively from an RNA-including body which is present in a sample such as biological sample and an environment sample, or in a sample such as a living body-derived sample obtained by separation of an RNA-including body and the like.
According to the present disclosure, by conducting inactivation of RNase in the sample and RNA extraction from inside of an RNA-including body in a single step, it is possible to amplify RNA which is present in the sample in a simple, stable, effective and rapid manner. Also by suppressing activity of a substance that inhibits nucleic acid synthesis, it is possible to amplify RNA which is present in the sample in a simpler, more stable, effective and rapid manner. As a result, it is possible to provide a method of detecting RNA in a sample in a simple, stable, effective and rapid manner.
According to the present disclosure, it is possible to provide a treating reagent which may be used in these methods.

## Claims

1. An RNA detection method, comprising the steps of:
obtaining a mixture under a heating condition comprising a heating time of 1 second to 60 minutes at a temperature of 60°C to 100°C,
said mixture comprising: a sample comprising an RNA virus and RNase, and an alkaline treating reagent comprising at least a reducing agent and a chelate agent to chelate a bivalent metal ion, and having pH of 8.1 or higher, said reducing agent comprised in the mixture in a concentration of 2.5mM to 25mM,
conducting inactivation of the RNase and extraction of RNA from the RNA virus by keeping the mixture under the heating condition specified above, thereby obtaining a treated sample liquid comprising extracted RNA, and
conducting RNA amplification reaction by mixing the treated sample liquid and an amplification reaction solution to obtain an amplification product, and
detecting the amplification product.

2. The RNA detection method according to claim 1, wherein the treating reagent comprises an alkaline buffer selected from the group consisting of a Tris buffer solution, a Good buffer solution, a borate buffer solution, and a carbonate buffer solution.

3. The RNA detection method according to claim 1, wherein the treating reagent comprises an alkaline substance selected from the group consisting of hydroxide, ammonia and amine.

4. The RNA detection method according to claim 1, wherein the reducing agent is a thiol type reducing agent.

5. The RNA detection method according to claim 1, wherein the sample is selected from the group consisting of a biological sample, a living body-derived sample, an environment sample, and an environment-derived sample.

6. The RNA detection method according to claim 1, wherein the sample is selected from the group consisting of an excrement sample and an excrement-derived sample.

7. The RNA detection method according to claim 1, wherein the RNA virus is selected from the group consisting of retrovirus, norovirus (SRSV), rotavirus, and hepatitis C virus (HCV).

8. The RNA detection method according to claim 7, wherein when the RNA virus is retrovirus, the retrovirus is AIDS virus (HIV).

9. The RNA detection method according to claim 1, wherein the RNA is mRNA.

10. An RNA detection method comprising the steps of:
mixing a sample comprising an RNA virus and RNase in a solution comprising at least a reducing agent and a chelate agent to chelate a bivalent metal ion to obtain a mixture so that said reducing agent is comprised in a concentration of 2.5mM to 25mM;
adjusting pH at 25°C of the mixture of the sample and the reducing agent to 8.1 or higher;
subjecting the mixture having adjusted pH to a heating condition comprising a heating time of 1 second to 60 minutes at a temperature of 60°C to 100°C, to conduct inactivation of the RNase and extraction of RNA from the RNA virus, thereby obtaining a treated sample liquid comprising extracted RNA, and
conducting RNA amplification reaction by mixing the treated sample liquid and an amplification reaction solution to obtain an amplification product, and
detecting the amplification product.

## Patentansprüche

1. RNA-Nachweisverfahren, umfassend die Schritte:
Erhalt einer Mischung unter einer Erhitzungsbedingung, welche eine Erhitzungszeit von 1 Sekunde bis 60 Minuten und eine Temperatur von 60°C bis 100°C umfasst,
wobei die Mischung umfasst: eine Probe, welche ein RNA-Virus und RNase enthält, und ein alkalisches Behandlungsreagenz, welches wenigstens ein Reduktionsmittel und ein Chelatmittel zur Chelation eines zweiwertigen Metallions umfasst und einen pH-Wert von 8,1 oder höher aufweist, wobei das Reduktionsmittel in der Mischung in einer Konzentration von 2,5 mM bis 25 mM enthalten ist,
Durchführen einer Inaktivierung der RNase und Extraktion von RNA aus dem RNA-Virus durch Halten der Mischung unter der oben spezifizierten Erhitzungsbedingung zum Erhalt einer behandelten Probenflüssigkeit, welche extrahierte RNA enthält, und
Durchführen einer RNA-Amplifikationsreaktion durch Mischen der behandelten Probenflüssigkeit und einer Amplifikationsreaktionslösung zum Erhalt eines Amplifikationsprodukts und
Detektieren des Amplifikationsprodukts.

2. RNA-Nachweisverfahren gemäß Anspruch 1, wobei das Behandlungsreagenz einen alkalischen Puffer, der ausgewählt ist aus der Gruppe bestehend aus einer Tris-Pufferlösung, einer Good-Pufferlösung, einer Boratpufferlösung und einer Carbonatpufferlösung, umfasst.

3. RNA-Nachweisverfahren gemäß Anspruch 1, wobei das Behandlungsreagenz eine alkalische Substanz, die ausgewählt ist aus der Gruppe bestehend aus Hydroxid, Ammoniak und Amin, umfasst.

4. RNA-Nachweisverfahren gemäß Anspruch 1, wobei das Reduktionsmittel ein Reduktionsmittel vom Thiol-Typ ist.

5. RNA-Nachweisverfahren gemäß Anspruch 1, wobei die Probe ausgewählt ist aus der Gruppe bestehend aus einer biologischen Probe, einer behandelten Probe vom lebenden Körper, einer Umweltprobe und einer behandelten Umweltprobe.

6. RNA-Nachweisverfahren gemäß Anspruch 1, wobei die Probe ausgewählt ist aus der Gruppe bestehend aus einer Exkrementprobe und einer behandelten Exkrementprobe.

7. RNA-Nachweisverfahren gemäß Anspruch 1, wobei das RNA-Virus ausgewählt ist aus der Gruppe bestehend aus Retrovirus, Norovirus (SRSV), Rotavirus und Hepatitis C-Virus (HCV).

8. RNA-Nachweisverfahren gemäß Anspruch 7, wobei, wenn das RNA-Virus ein Retrovirus ist, das Retrovirus ein AIDS-Virus (HIV) ist.

9. RNA-Nachweisverfahren gemäß Anspruch 1, wobei es sich bei der RNA um mRNA handelt.

10. RNA-Nachweisverfahren, umfassend die Schritte:
Mischen einer Probe, die ein RNA-Virus und RNase enthält, in einer Lösung, die wenigstens ein Reduktionsmittel und ein Chelatmittel zur Chelation eines zweiwertigen Metallions umfasst, zum Erhalt einer Mischung, so dass das Reduktionsmittel in einer Konzentration von 2,5 mM bis 25 mM enthalten ist;
Einstellen des pH-Werts bei 25 °C der Mischung aus der Probe und dem Reduktionsmittel auf 8,1 oder höher;
Aussetzten der Mischung mit eingestelltem pH-Wert einer Erhitzungsbedingung, welche eine Erhitzungszeit von 1 Sekunde bis 60 Minuten und eine Temperatur von 60 °C bis 100 °C umfasst, zur Durchführung einer Inaktivierung der RNase und Extraktion von RNA aus dem RNA-Virus zum Erhalt einer behandelten Probenflüssigkeit, welche extrahierte RNA enthält, und
Durchführen einer RNA-Amplifikationsreaktion durch Mischen der behandelten Probenflüssigkeit und einer Amplifikationsreaktionslösung zum Erhalt eines Amplifikationsprodukts und
Detektieren des Amplifikationsprodukts.

## Revendications

1. Procédé de détection d'ARN comprenant les étapes :
- obtenir un mélange dans des conditions de chauffage comprenant un temps de chauffage allant de 1 seconde à 60 minutes à une température de 60°C à 100°C,
ledit mélange comprenant : un échantillon comprenant un virus à ARN et de la RNase, et un réactif traitant alcalin comprenant au moins un agent réducteur et un agent chélatant afin de chélater un ion métallique bivalent, et ayant un pH de 8,1 ou plus, ledit agent réducteur étant présent dans le mélange à une concentration de 2,5 mM à 25 mM,
- effectuer l'inhibition de la RNase et l'extraction d'ARN du virus à ARN en maintenant le mélange dans les conditions de chauffage spécifiées ci-dessus et obtenir ainsi un échantillon liquide traité comprenant l'ARN extrait, et
- effectuer la réaction d'amplification de l'ARN en mélangeant l'échantillon liquide traité et une solution réactionnelle d'amplification afin d'obtenir un produit d'amplification, et
- détecter le produit d'amplification.

2. Procédé de détection d'ARN selon la revendication 1, dans lequel le réactif traitant comprend un tampon alcalin choisi dans le groupe constitué d'une solution tampon Tris, d'une solution tampon de Good, d' une solution tampon borate et d' une solution tampon carbonate.

3. Procédé de détection d'ARN selon la revendication 1, dans lequel le réactif traitant comprend une substance alcaline choisie dans le groupe constitué de l'hydroxyde, de l'ammoniaque et de l'amine.

4. Procédé de détection d'ARN selon la revendication 1, dans lequel l'agent réducteur est un agent réducteur de type thiol.

5. Procédé de détection d'ARN selon la revendication 1, dans lequel l'échantillon est choisi dans le groupe constitué d'un échantillon biologique, d'un échantillon dérivé d'un organisme vivant, d'un échantillon environnemental et d'un échantillon dérivé de l'environnement.

6. Procédé de détection d'ARN selon la revendication 1, dans lequel l'échantillon est choisi dans le groupe constitué d'un échantillon d'excrément et d'un échantillon dérivé d'un excrément.

7. Procédé de détection d'ARN selon la revendication 1, dans lequel le virus à ARN est choisi dans le groupe constitué des rétrovirus, des norovirus (SRSV), des rotavirus et du virus de l'hépatite C (VHC).

8. Procédé de détection d'ARN selon la revendication 7, dans lequel le virus à ARN est un rétrovirus et le rétrovirus est le virus du SIDA (VIH).

9. Procédé de détection d'ARN selon la revendication 1, dans lequel l'ARN est l'ARNm.

10. Procédé de détection d'ARN comprenant les étapes :
- mélanger un échantillon comprenant un virus à ARN et de la RNase dans une solution comprenant au moins un agent réducteur et un agent chélatant afin de chélater un ion métallique bivalent afin d'obtenir un mélange de manière à ce que ledit agent réducteur soit présent dans le mélange à une concentration de 2,5 mM à 25 mM ;
- ajuster à 25°C le pH du mélange de l'échantillon et de l'agent réducteur à 8,1 ou plus ;
- soumettre le mélange ayant un pH ajusté à une condition de chauffage comprenant un temps de chauffage allant de 1 seconde à 60 minutes à une température de 60°C à 100 °C, afin d' effectuer l'inhibition de la RNase et l'extraction de l'ARN du virus à ARN, et obtenir ainsi un échantillon liquide traité comprenant l'ARN extrait ; et
- effectuer la réaction d'amplification de l'ARN en mélangeant l'échantillon liquide traité et une solution réactionnelle d'amplification afin d'obtenir un produit d'amplification, et
- détecter le produit d'amplification.
